# EUROPEAN PATENT SPECIFICATION

(11) **EP 0 068 466 B1**
(45) Date of publication and mention of the grant of the patent: **14.01.1987**
(21) Application number: 82105647.0
(22) Date of filing: 25.06.1982
(51) Int. Cl.: C07D 417/14, C07D 403/14, C07D 403/12, C07D 409/12, C07D 413/14, C07D 413/12, C07D 401/12, C07D 205/08, A61K 31/505, A61K 31/495, A61K 31/44

(54) **4-Aryl or heteroaryl thio-2-oxo-1-azetidinesulfonic acid salts and process for their preparation**
4-Aryl- oder Heteroaryl-thio-2-oxo-1-azetidinsulfonsäuresalze und Verfahren zu deren Herstellung
Sels (aryl ou hétéroaryl) thio-4 oxo-2 azétidine-sulfoniques-1 et leur procédé de préparation

(30) Priority: 29.06.1981 US 278131
(43) Date of publication of application: 05.01.1983
(73) Proprietor: E.R. Squibb & Sons, Inc., Princeton, N.J. 08540-4000 (US)
(72) Inventor: Treuner, Uwe D., D-8400 Regensburg (DE); Denzel, Theodor, D-8400 Regensburg (DE); Breuer, Hermann, D-8411 Schoenhofen (DE)
(74) Representative: VOSSIUS & PARTNER

## Description

The present invention relates to 4-aryl or heteroaryl-thio-2-oxo-1-azetidine-sulfonic acid salts 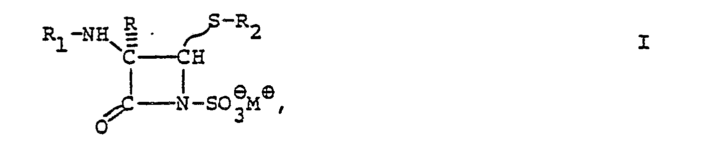 In formula I, and throughout the specification, the symbols are as defined below.
R is hydrogen or methoxy;
R₁ is acyl;
R₂ is aryl, 1H-tetrazol-5-yi, 1-alkyl-1H-tetrazol-5-yl, 1,3,4-thiadiazol-2-yl, 5-alkyl-1,3,4-thiadiazol-2-yl, 1,2,4-thiadiazol-5-yl, 3-alkyl-1,2,4-thiadiazol-5-yl, 1,3,4-oxadiazol-2-yl, 5-alkyl-1,3,4-oxadiazol-2-yl, 1,2,4-oxadiazol-5-yl, 3-alkyl-1,2,4-oxadiazol-5-yl, 2-pyridinyl, 3-pyridinyl, 4-pyridinyl, 2-pyrimidinyl, benzothiazol-2-yl, 4H-1,2,3-triazol-5-yl, 5-alkyl-H-1,2,3-triazol-5-yl, 1-H-imidazol-2-yl, or 1-alkyl-1H-imidazol-2-yl; and
M⊕ is hydrogen or a cation, with the proviso that if M° is hydrogen the R₁ group contains a basic function. These compounds belong to the class of β-lactams.
Listed below are definitions of various terms used to describe the β-lactams of this invention. These definitions apply to the terms as they are used throughout the specification (unless they are otherwise limited in specific instances) either individually or as part of a larger group.

The term "acyl" includes all acyl groups derived from an organic acid (i.e., a carboxylic acid) by removal of the hydroxyl group which acyl groups have been used in the past to acylate β-lactam antibiotics including 6-aminopenicillanic acid and derivatives and 7-aminocephalosporanic acid and derivatives; see, for example, Cephalosporins and Penicillins, edited by Flynn, Academic Press (1972), German Offenlegungsschrift 2,716,677, published October 10, 1978, Belgian patent 867,994, published December 11,1978, United States patent 4,152,432, issued May 1, 1979, United States patent 3,971,778, issued July 27, 1976, United States patent 4,172,199, issued October 23, 1979, and British patent 1,348,894, published March 27,1974. The portions of these references describing various acyl groups are incorporated herein by reference. The following list of acyl groups is presented to further exemplify the term "acyl"; it should not be regarded as limiting that term. Exemplary acyl groups are:
(a) Aliphatic groups having the formula 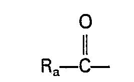 wherein Rₐ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.
(b) Carbocyclic aromatic groups having the formula 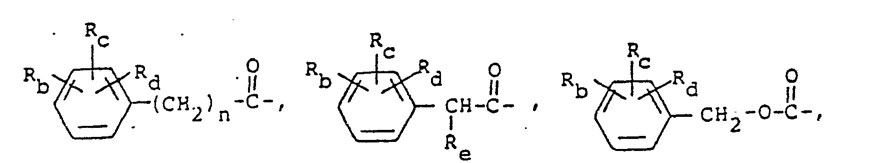 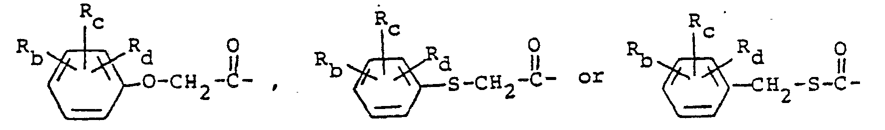 wherein n is 0, 1, 2 or 3; R_{b}, R_{c}, and R_{d} each is independently hydrogen, halogen, hydroxyl, nitro amino, cyano, trifluoromethyl, alkyl of 1 to 4 carbon atoms, alkoxy of 1 to 4 carbon atoms or aminomethyl; and Rₑ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula 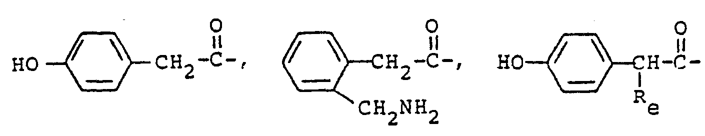 (Rₑ is preferably a carboxyl salt or sulfo salt) and 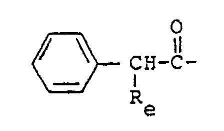 (Rₑ is preferably a carboxyl salt or sulfo salt).

(c) Heteroaromatic groups having the formula 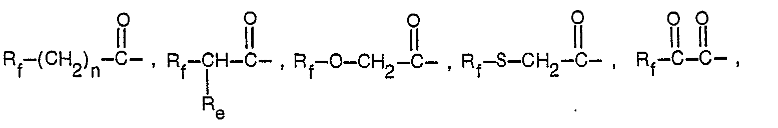 wherein n is 0, 1, 2 or 3; Rₑ is as defined above; and R_{f} is a substituted or unsubstituted 5-, 6- or 7- membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, pyrimidinyl and tetrazolyl. Exemplary substituents are halogen, hydroxyl, nitro, amino, cyano, trifluoromethyl, alkyl of 1 to 4 carbon atoms, alkoxy of 1 to 4 carbon atoms, or

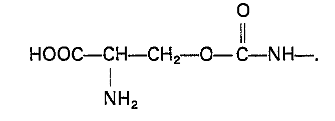

Preferred heteroaromatic acyl groups include those groups of the above formulas wherein R_{f} is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.
(d) [[(4-(Substituted-2,3-dioxo-1-piperazinyl)carbonyl]amino]arylacetyl groups having the formula 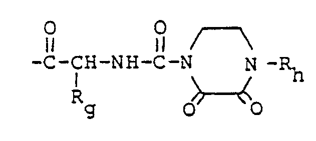 wherein Rg is an aromatic group (including carbocyclic aromatics such as those of the formula 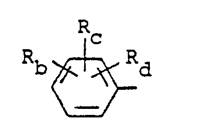 and heteroaromatics as included within the definition of R_{f}); and Rₕ is alkyl, substituted alkyl (wherein the alkyl group is substituted with one or more halogen, cyano, nitro, amino or mercapto groups), arylmethyleneamino (i.e., -N=CH-Rg wherein Rg is as defined above), arylcarbonylamino (i.e., 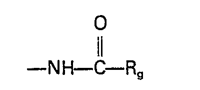 wherein Rg is as defined above) or alkylcarbonylamino.
Preferred [[(4-substituted-2,3-dioxo-1-piperazinyl)carbonyl]amino]arylacetyl groups include those wherein Rₕ is ethyl, phenylmethyleneamino or 2-furylmethyleneamino.
(e) (Substituted oxyimino)arylacetyl groups having the formula 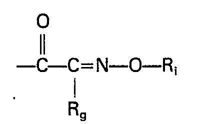 wherein Rg is as defined above and R is hydrogen, alkyl, cycloalkyl, alkylaminocarbonyl, arylaminocarbonyl (i.e., 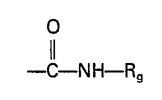 wherein Rg is as defined above) or substituted alkyl (wherein the alkyl group is substituted with 1 or more halogen, cyano, nitro, amino, mercapto, alkylthio, aromatic group (as defined by Rg), carboxyl (including salts thereof), amido, alkoxycarbonyl, phenylmethoxycarbonyl, diphenylmethoxycarbonyl, hydroxy- alkoxyphosphinyl, dihydroxyphosphinyl, hydroxy(phenylmethoxy)phosphinyl, or dialkoxyphosphinyl substituents).

Preferred (substituted oxyimino)arylacetyl groups include those wherein R_{g} is 2-amino-4-thiazolyl. Also preferred are those groups wherein Rᵢ is methyl, ethyl, carboxymethyl, 1-carboxy-1-methylethyl or 2,2,2-trifluoroethyl.
(f) (Acylamino)arylacetyl groups having the formula 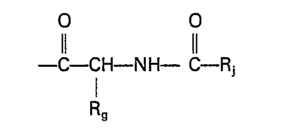 wherein R_{g} is as defined above and Rⱼ is 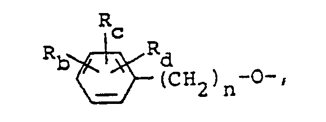 amino, alkylamino, (cyanoalkyl)amino, amido, alkylamido, (cyanoalkyl)amido,

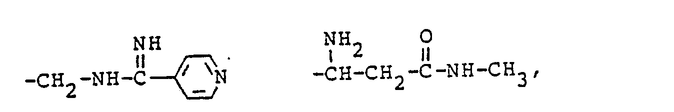

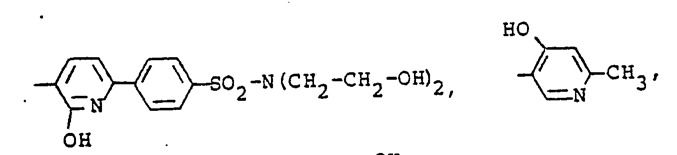

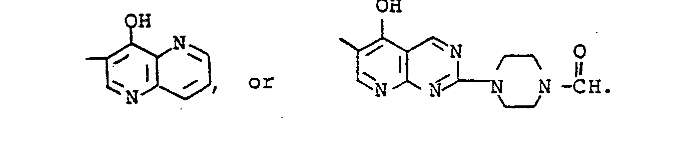

Preferred (acylamino)arylacetyl groups of the above formula include those groups wherein Rⱼ is amino or amido. Also preferred are those groups wherein R_{g} is phenyl or 2-thienyl.
(g) [[[3-Substituted-2-oxo-1-imidazolidinyl]carbonyl]amino]arylacetyl groups having the formula 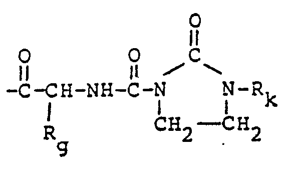 wherein Rg is as defined above and Rₖ is hydrogen, alkylsulfonyl, arylmethyleneamino (i.e., -N=CH-R₉ wherein Rg is as defined above), 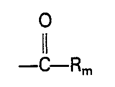 (wherein Rₘ is hydrogen, alkyl or halogen substituted alkyl), aromatic group (as defined by Rg above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein Rg is phenyl or 2-thienyl. Also preferred are those groups wherein Rₖ is hydrogen, methylsulfonyl, phenylmethyleneamino or 2-furylmethyleneamino.

The terms "alkyl" and "alkoxy" refer to both straight and branched chain groups. Those groups having 1 to 10 carbon atoms are preferred.

The terms "alkanoyl" and "alkenyl" refer to both straight and branched chain groups. Those groups having 2 to 10 carbon atoms are preferred.

The terms "cycloalkyl" and "cycloalkenyl" refer to groups having 3, 4, 5, 6 or 7 carbon atoms.

The term "cation", as used throughout the specification, refers to any positively charged atom or group of atoms. The " 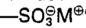 " substituent on the nitrogen atom of the β-lactams of this invention encompass all sulfonic acid salts. Pharmaceutically acceptable salts are, of course, preferred, although other salts are also useful in purifying the products of this invention or as intermediates for the preparation of pharmaceutically acceptable salts. The cationic portion of the sulfonic acid salts of this invention can be obtained from either organic or inorganic bases. Such cationic portion includes, but is not limited to, the following ions: ammonium; substituted ammonium, such as alkylammonium (e.g., tetra-n-butylammonium, referred to hereinafter as tetrabutylammonium); alkali metal, such as lithium, sodium and potassium; alkaline earth metal, such as calcium and magnesium; pyridinium; dicyclohexylammonium; hydrabaminium; benzathinium; N-methyl-D-glucaminium.

The term "aryl" refers to phenyl and phenyl substituted with 1, 2 or 3 amino (-NH₂), halogen, hydroxyl, trifluoromethyl, alkyl (of 1 to 4 carbon atoms) or alkoxy (of 1 to 4 carbon atoms) groups.

As set forth in formula I, and in the definitions following formula 1, M⁹ can be hydrogen. Such compounds are often referred to in the art as "inner salts" by virtue of a positive and negative charge in the molecule.

This invention is directed to those β-lactams which have been described above, wherein the stereochemistry at the chiral center in the 3-position of the β-lactam nucleus is the same as the configuration at the carbon atom in the 6-position of naturally occurring penicillins (e.g., penicillin G) and as the configuration at the carbon atom in the 7-position of naturally occurring cephamycins (e.g., cephamycin C).

The β-lactams of formula I have activity against a range of gram-negative and gram-positive organisms. The compounds of this invention can be used as agents to combat bacterial infections (including urinary tract infections and respiratory infections) in mammalian species, such as domesticated animals (e.g., dogs, cats, cows, horses, and the like) and humans.

For combating bacterial infections in mammals a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with the novel β-lactams of this invention. Such method of administration include oral, intravenous, intramuscular, and as a suppository.

The compounds of formula I can be prepared from an azetidine having the formula 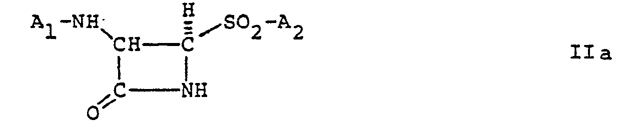 or 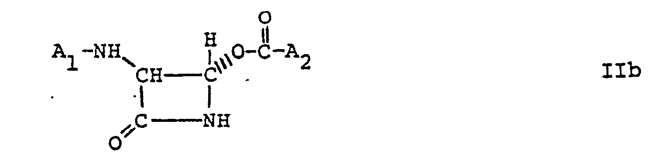 wherein A₁ is a nitrogen protecting group, e.g., triphenylmethyl, and A₂ is an alkyl or aryl group. Reaction of an azetidine of formula Ila or Ilb with a thiolate having the formula 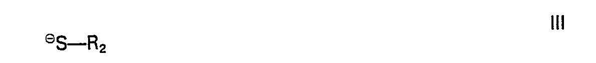 yields a mixture of diastereomers of the formulas 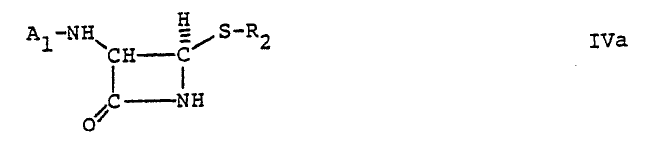 and

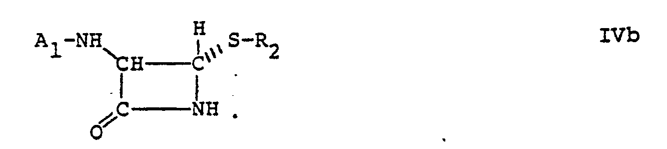

Separation of the above diastereomers can be accomplished using conventional chromatographic or fractional crystallization techniques.

The addition of a sulfo ( 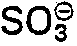 ) group to the 1-position of a compound of formula IVa or IVb or a corresponding compound wherein A₁ has been replaced by the R₁-group yields the corresponding compound having the formula

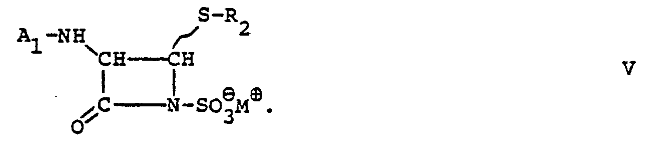

Introduction of the sulfo substituent can be accomplished by reacting a compound of formula IVa or IVb with a complex of pyridine and sulfur trioxide. The reaction can be run in an organic solvent or in a mixture of organic solvents, preferably a mixture of a polar solvent such as dimethylformamide and a halogenated hydrocarbon such as dichloromethane. This reaction yields a compound of formula V wherein M^{I} is pyridinium ion. Instead of using a pre-formed complex of pyridine and sulfur trioxide, the complex can be formed in situ, e.g., using chlorosulfonyltrimethylsilyl ester and pyridine as reagents. Alternatively, a complex of dimethylformamide-sulfur trioxide, 2-picoline-sulfur trioxide or 2,6-lutidine sulfur trioxide can be used.

Using conventional techniques (e.g., ion-exchange resins, crystallization, or ion-pair extraction) the pyridinium salt formed by the above procedure can be converted to other salts. These techniques can also be used to convert the products of formula I, or any of the intermediates described herein, to other salts.

A second method for introducing the sulfo group to the 1-position of an azetidine of formula lVa or IVb comprises first silylating the compound and then subjecting the silylated compound to a silyl interchange reaction. Exemplary silylating agents are monosilyltriflouoroacetamide, trimethylsilylchloride/ triethylamine, and bis-trimethylsilyltrifluoroacetamide, and an exemplary reagent useful for the silyl interchange reaction is trimethylsilyl chlorosulfonate.

Deprotection of an azetidine of formula V yields a zwitterion having the formula

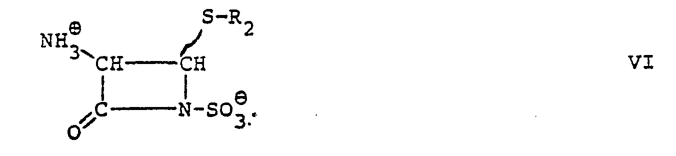

The deprotection techniques used are conventional, and will depend on the particular protecting group (A₁) present. Treatment with acid (e.g., formic acid or trifluoroacetic acid) cleaves a triphenylmethyl or a t-butoxycarbonyl protecting group. A benzyloxycarbonylamino protecting group can be cleaved by treatment with trimethylsilyl iodide. Treatment with phosgene or phosphorous pentachloride cleaves an amide protecting group. The zwitterions of formula VI are novel intermediates, and as such, constitute an integral part of this invention.

Conventional acylation techniques can be used to prepare the products of formula I (wherein R is hydrogen) from a zwitterion of formula VI. Exemplary acylation techniques include reaction with a carboxylic acid (R₁-OH), or corresponding carboxylic acid halide or carboxylic acid anhydride. The reaction with a carboxylic acid proceeds most readily in the presence of a carbodiimide and a substance capable of forming an active ester in situ such as N-hydroxybenzotriazole. In those instances wherein the acyl group (R₁) contains reactive functionality (such as amino or carboxyl groups) it may be necessary to first protect those functional groups, then carry out the acylation reaction, and finally deprotect the resulting product.

Variations of the above described synthetic route (some of which are described in the examples presented below) will be apparent to the practitioner of this invention. For example, a compound of formula IVa or IVb can be deprotected and then protected with a different amino protecting group before proceeding with the remaining synthetic procedure.

The (3-lactams of formula I wherein R is methoxy can be prepared from the corresponding compound of formula I wherein R is hydrogen. Halogenation of the amide nitrogen of a non-methoxylated compound of formula I yields, in situ, an intermediate having the formula

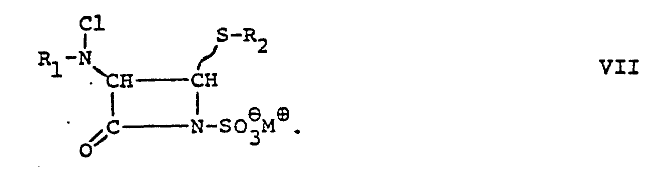

Reaction of an intermediate of formula VII with a methoxylating agent, e.g., an alkali metal methoxide yields a product of formula I wherein R is methoxy. The reaction can be run in an organic solvent, e.g., a polar organic solvent such as dimethylformamide, at a reduced temperature.

An alternative synthesis for preparing the compounds of formula I wherein R is methoxy comprises first alkoxylating a compound of formula IVa or IVb wherein A↑NH is a carbamate (e.g., A₁ is benzyloxycarbonyl) and then introducing a sulfo group in the 1-position of the resulting compound. Deprotection and acylation, using the procedures described above, yields the products of formula I wherein R is methoxy.

The following examples are specific embodiments of this invention.

### Example 1

### (3R-trans)-3-Amino-4-[(1-methyl-1H-tetrazol-5-yl]thio]-2-oxo-1-azetidinesulfonic acid, inner salt

### Method I

### A) (3R-cis)-4-[(1-methyl-1H-tetrazol-5-yl)thio]-3-[(triphenylmethyl)amino]-2-azetidinone and (3R-trans)-4-[(1-methyl-1H-tetrazol-5-yl)thio]-3-[(triphenylmethyl)amino]-2-azetidinone

(cis)-[2-Oxo-3-[(triphenylmethyl)amino]-4-azetidinyl]methyl sulfone (2.04 g) is dissolved in 30 ml of methanol, 0.2 g of 1,4,7,10,13, 16-hexaoxacyclooctadecane is added, and with stirring, a solution of 0.8 g of the sodium salt of 5-mercapto-1-methyl-1H-tetrazole in 20 ml of methanol is added dropwise at a temperature of 60°C. Stirring is continued for 3 hours. The reaction solution is poured into 200 ml of ice-water yielding 2.6 g of crystalline crude product. Chromatography on silica, eluting with dichloromethane- ethyl acetate (9:1), yields 0.7 g of the cis isomer, 0.9 g of the trans isomer, and 0.4 g of a mixture of isomers.

### B) (3R-trans)-4-[(1-methyl-1H-tetrazol-5-yl)thio]-2-oxo-3-[(triphenylmethyl)amino]-1-azetidinesulfonic acid, tetrabutylammonium salt

(3R-trans)-4-[(1-methyl-1H-tetrazol-5-yl)thio]-3-[(triphenylmethyl)amino]-2-azetidinone (4.25 g) and 3.2 g of a complex of pyridine-sulfur trioxide are dissolved in dimethylformamide and stirred for 48 hours at room temperature. The solution is poured into 200 ml of ice-water, 3.26 g of tetrabutylammonium hydrogen sulfate are added and the pH is adjusted to pH 5.5 with 1 N potassium hydroxide. Ion pair extraction with dichloromethane yields 6.2 g of crude title compound as an oil.

### C) (3R-trans)-3-Amino-4-[(1-methyl-1H-tetrazol-5-yl)thio]-2-oxo-1-azetidinesulfonic acid, inner salt

(3R-trans-)-4-[(1-methyl-1H-tetrazol-5-yl)thio]-2-oxo-3-[(triphenylmethyl)amino]-1-azetidinesulfonic acid, tetrabutylammonium salt (6 g) are stirred in 20 ml of formic acid and dichloromethane (9:1) for about 1 hour. The solution is poured into 200 ml of ether, precipitating the title compound as a powder, melting point 112°C, dec.

### Method II

### A) (3R-trans-)-3-Amino-4-[(1-methyl-1H-tetrazol-5-yl)thio]-2-azetidinone,p-toluenesulfonate

(3R-trans-)-4-[(1-Methyl-1H-tetrazol-5-yl)thio]-3-[(triphenylmethyl)amino]-2-azetidinone (3.5 g; see method I, part A) and 1.5 g of p-toluenesulfonic acid are stirred in 20 ml of dichloromethane at 0°C for 90 minutes. Ether is adding, causing the title compound to precipitate; melting point 137°C, dec.

### B) (3R-trans-)-4-[(1-methyl-1H-tetrazol-5-yl)-thio]-3-[[(1,1-dimethylethyl)carbonyl]amino]-2-azetidinone

(3Rctrans-)-3-Amino-4-[(1-methyl-1H-tetrazol-5-yl)thio]-2-azetidinone, p-toluenesulfonate (2.0 g) and 1.08 g of triethylamine are dissolved in 15 ml of t-butanol and 1.4 g of di-t-butylpyrocarbonate in tetrahydrofuran are added. After stirring for 8 hours at 5°C, the solvent is distilled off and the residue is dissolved in 50 ml of ethyl acetate. Extraction with aqueous citric acid and water yields the title compound from the organic phase; melting point 105°C, dec.

### C) (3R-trans-)-3-Amino-4-[(1-methyl-1H-tetrazol-5-yl)thio]-2-oxo-1-azetidinesulfonic acid, inner salt

(3R-trans-)-4-[(1-methyl-1H-tetrazol-5-yl)thio]-3-[[(1,1-dimethylethyl)carbonyl]amino]-2-azetidinone (300 mg) and 320 mg of a complex of pyridine and sulfur trioxide are dissolved in 10 ml of dimethylformamide and stirred for 12 hours at 50°C. The solution is poured into 80 ml of ice-water, 370 mg of tetrabutylammonium hydrogen sulfate is added and the pH is adjusted to 5.5 with 1 N potassium hydroxide. Extraction with dichloromethane yields an oily residue from the organic phase. This is stirred for 1 hour in 10 ml of formic acid/dichloromethane (9:1) and poured into 40 ml of ether, yielding 110 mg of the title compound.

### Example 2

### [(3R-trans-)(Z)]-3-[[[(2-Amino-4-thiazolyl)methoxyimino]acetyl]amino]-4-[(1-methyl-1H-tetrazol-5-yl)thio]-2-oxo-1-azetidinesulfonic acid, potassium salt

(3R-trans-)-3-Amino-4-[(1-methyl-1H-tetrazol-5-yl)thio]-2-oxo-1-azetidinesulfonic acid, inner salt (268.2 mg; see example 1), 202 mg of (Z)-2-amino-a-(methoxyimino)-4-thiazoleacetic acid, 101 mg of triethylamine and 175 mg of N-hydroxybenzotriazole are dissolved in 10 ml of dimethylformamide. At 0°C a solution of 206 mg of dicyclohexylcarbodiimide in 3 ml of tetrahydrofuran is added dropwise. After stirring for 12 hours at room temperature, the solvent is distilled off and the residue is dissolved in acetone. Filtration, followed by the addition of 340 mg of potassium perfluorobutane sulfonate to the filtrate yields 0.3 g of crude product. Column chromatography on HP20 using water/acetone (9:1) as eluent yields the title compound, melting point 176-180°C.

### Example 3

### [(3R-trans-)(R^{*})]-3_{-[[[[}(4-ethyl-2,3-dioxo-1-piperazinyl)carbonyl]amino]phenylacetyl]amino]-2-oxo-4-[(1-methyl-1H-tetrazol-5-yI)thio]-1-azetidinesulfonic acid, potassium salt

Following the procedure of example 2, but substituting (R)-a-[[(4-ethyl-2,3-dioxo-1-piperazinyl)carbonyl]amino]benzeneacetic acid for (Z)-2-amino-a-(methoxyimino)-4-thiazoleacetic acid, yields the title compound, melting point 184°C.

### Example 4

### (3R-trans-)-4-[(1-Methyl-1H-tetrazol-5-yl)thio]-2-oxo-3-[[(phenylmethoxy)carbonyl]amino]-1- azetidinesulfonic acid, potassium salt

A) (3R-trans-)-4-[(1-Methyl-1H-tetrazol-5-yl)thio]-3-[[(phenylmethoxy)carbonyl]amino]-2-azetidinone (3R-trans-)-4-(Acetyloxy)-3-[[(phenylmethoxy)carbonyl]amino]-2-azetidinone (1.4 g) is dissolved in 20 ml of dry dimethylformamide at a temperature of 50°C. The sodium salt of 5-mercapto-1-methyl-1H tetrazole (0.8 g) is added to the solution and the resulting mixture is stirred for 20 minutes. Water (300 ml) is added and the mixture is filtered yielding the title compound. Recrystallization from ethyl acetate yields 1.2 g of the title compound, melting point 194°C, dec.

### B) (3R-trans-)-4-[(1-Methyl-1H-tetrazol-5-yl)thio]-2-oxo-3-[[(phenylmethoxy)carbonyl]amino]-1- azetidinesulfonic acid, potassium salt

(3R-trans-)-4-[(1-Methyl-1H-tetrazol-4-yl)thio]-3-[[(phenylmethoxy)carbonyl]amino-2-azetidinone (1.7 g) is dissolved in 30 ml of acetonitrile (dried) and 3 ml of monosilyltrifluoroacetamide is added; the solution is maintained at 50°C for 2 hours. The mixture is evaporated to remove excess monosilyltrifluoroacetamide. The remaining oil is dissolved in 15 ml of acetonitrile, cooled to -10°C and 6 mmol of trimethylsilyl chlorosulfonate is added; stirring is continued for 30 minutes. A 2N solution (5 ml) of potassium 2-ethylhexanoate in n-butanol is added and the title compound precipitates. The precipitate is filtered off, dissolved in 10 mi of dibasic potassium phosphate/potassium hydroxide buffer (pH 5.0) and chromatographed on HP20 resin, eluting with water. After freeze-drying, 0.9 g of the title compound is obtained; melting point 143°C, dec.

### Examples 5-14

Following the procedures of examples 1 (method I) and 2, but substituting the sodium salt of the compound listed in column I for the sodium salt of 5-mercapto-1-methyl-1H-tetrazole and the compound listed in column II for (Z)-2-amino-o-(methoxyimino)-4-thiazoleacetic acid, yields the compound listed in column III. Examples 11 and 12 require a deprotection step at the end of the reaction. Deprotection is accomplished in examples 11 and 12 by treatment of the protected compound with trifluoroacetic acid and anisole at -15°C.

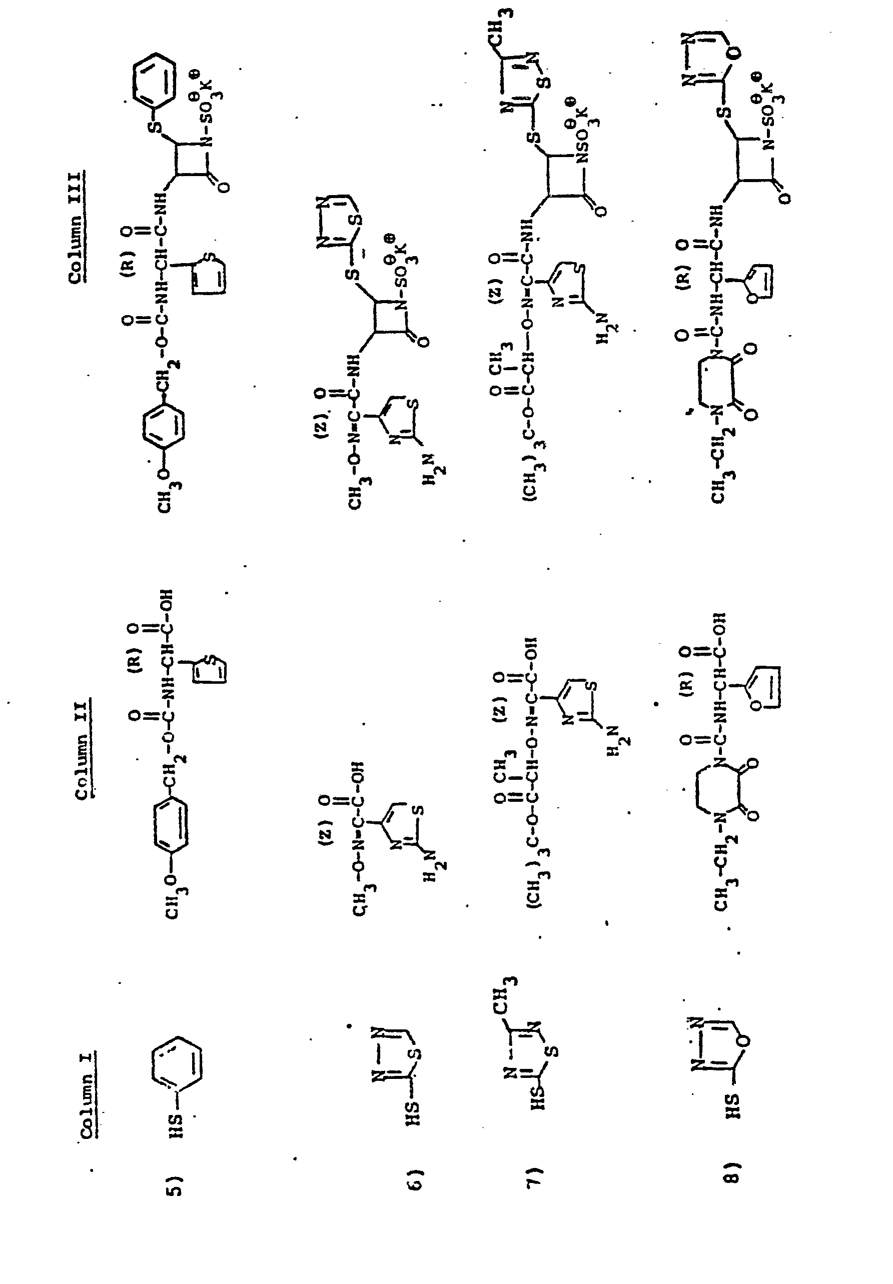

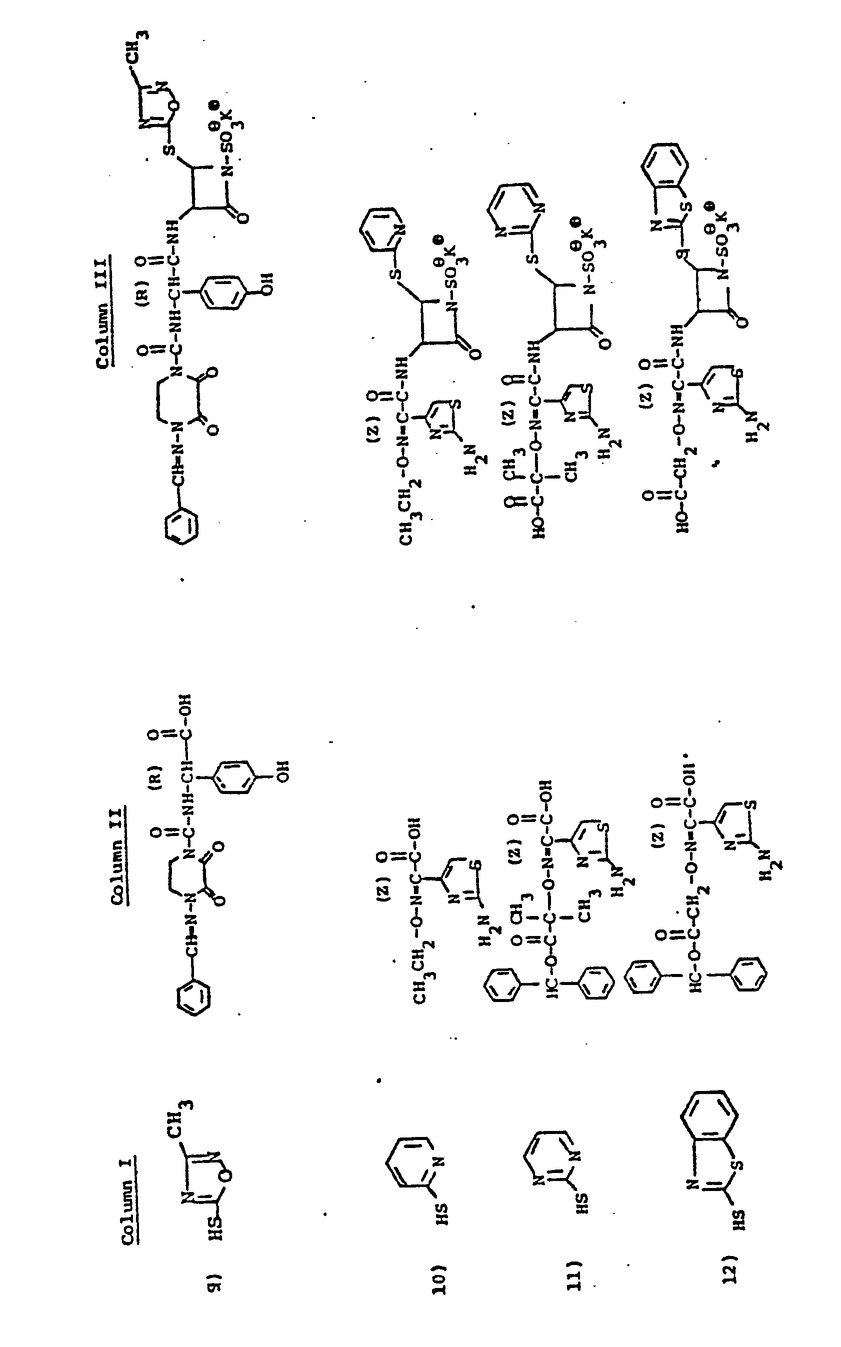

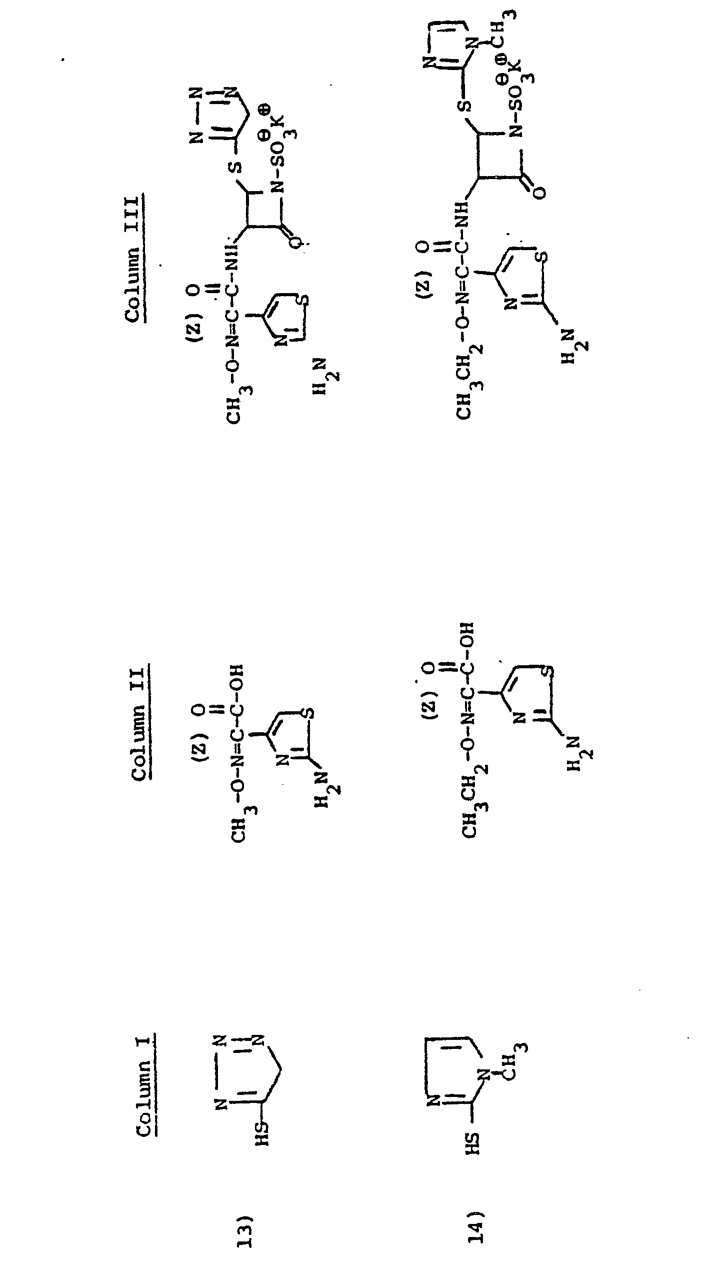

## Claims (Claims for the following Contracting State(s) : s: BE CH DE FR IT LI LU NL SE)

1. 4-Aryl or heteroarylthio-2-oxo-1-azetidinesulfonic acid salts having the formula 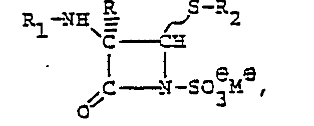 wherein
R is hydrogen or methoxy;
R₁ is an acyl group being known in conventional β-lactam antibiotics;
R₂ is phenyl, optionally substituted with 1, 2 or 3 amino, halogen, hydroxyl, trifluoromethyl, C₁-₄ alkyl or C₁₋₄ alkoxy groups, 1H-tetrazol-5-yl, 1-C₁₋₁₀ alkyl-1H-tetrazol-5-yl, 1,3,4-thiadiazol-2-yl, 5-C₁₋₁₀ alkyl-1,3,4-thiadiazol-2-yl, 1,2,4-thiadiazol-5-yl, 3-C₁₋₁₀ alkyl-1,2,4-thiadiazol-5-yl, 1,3,4-oxadiazol-2-yl, 5-C₁₋₁₀ alkyl-1,3,4-oxadiazol-2-yl, 1,2,4-oxadiazol-5-yl, 3-C₁₋₁₀ alkyl-1,2,4-oxadiazol-5-yl, 2-pyridinyl, 3-pyridinyl, 4-pyridinyl, 2-pyrimidinyl, benzothiazol-2-yl, 4H-1,2,3-triazol-5-yl, 5-C₁₋₁₀ alkyl-4H-1,2,3-triazol-5-yl, 1H-imidazol-2-yl, or 1-C₁₋₁₀ alkyl-1H-imidazol-2-yl; and

M⊕ is hydrogen or a cation, with the proviso that if M⊕ is hydrogen the R₁ group contains a basic function, those compounds being excluded which are described in EP 81 110 154.2 (Publication No. 53 815), namely
sodium (3R,4S)-3-[2-(2-chloroacetamidothiazol-4-yl)-2-methoxylminoacetamido]-4-phenylthio-2-oxo- azetidine-1-sulfonate,
sodium (3R,4S)-3-[2-(2-aminothiazol-4-yl)-2-methoxyiminoacetamido]-4-phenylthio-2-oxoazetidine-1-sulfonate,
sodium (3R,4S)-3-[2-(2-chloroacetamidothiazol-4-yl)-2-methoxyiminoacetamido]-4-(1-methyl-1H-tetrazol-5-yl)thio-2-oxoazetidine-1-sulfonate,
sodium (3R,4S)-3-[2-(2-aminothiazol-4-yl)-2-methoxyiminoacetamido]-4-(1-methyl-1H-tetrazol-5-yl)thio-2-oxoazetidine-1-sulfonate,
sodium (3R,4R)-3-[2-(2-chloroacetamido-thiazol-4-yl)-2-methoxyiminoacetamido]-4-(1-methyl-1H-tetrazol-5-yl)thio-2-oxoazetidine-1-sulfonate and
sodium (3R,4R)-3-[2-(2-aminothiazol-4-yl)-2-methoxyiminoacetamido]-4-(1-methyl-1H-tetrazol-5-yl)thio-2-oxoazetidine-1-sulfonate.

2. A compound in accordance with claim 1 having the formula

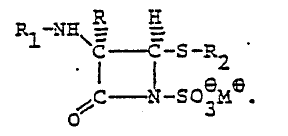

3. A compound in accordance with claim 1 having the formula

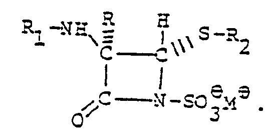

4. The compound in accordance with claim 1 [(3R-trans)(Z)]-[[[(2-amino-4-thiazolyl)methoxy- imino]acetyl]amino]-4-[(1-methyl-1H-tetrazol-5-yl)thio]-2-oxo-1-azetidinesulfonic acid, potassium salt.

5. The compound in accordance with claim 1 [(3R-trans)(R*)]-3-[[[[(4-ethyl-2,3-dioxo-1-piperazinyl)carbonyl]amino]phenylacetyl]amino]-2-oxo-4-[(1-methyl-1H-tetrazol-5-yl)thio]-1-azetidinesulfonic acid, potassium salt.

6. The compound in accordance with claim 1 (3R-trans)-4-[(1-methyl-1H-tetrazol-5-yl)thio]-2-oxo-3-[[(phenylmethoxy)carbonyl]amino]-1-azetidinesulfonic acid, potassium salt.

7. A compound having the formula 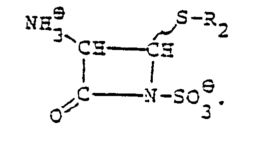 wherein R₂ is phenyl, optionally substituted with 1, 2 or 3 amino, halogen, hydroxyl, difluoromethyl, C₁₋₄ alkyl or C₁₋₄ alkoxy groups, 1H-tetrazol-5-yl, 1-C₁₋₁₀ alkyl-1H-tetrazol-5-yl, 1,3,4-thiadiazol-2-yl, 5-C₁₋₁₀ alkyl-1,3,4-thiadiazol-2-yl, 1,2,4-thiadiazol-5-yl, 3-C₁₋₁₀ alkyl-1,2,4-thiadiazol-5-yl, 1,3,4-oxadiazol-2-yl, 5-C₁₋₁₀ alkyl-1,3,4-oxadiazol-2-yl, 1,2,4-oxadiazol-5-yl, 3-C₁₋₁₀ alkyl-1,2,4-oxadiazol-5-yl, 2-pyridinyl, 3-pyridinyl, 4-pyridinyl, 2-pyrimidinyl, benzothiazol-2-yl, 4H-1,2,3-triazol-5-yl, 5-C₁₋₁₀ alkyl-4H-1,2,3-triazol-5-yl, 1H-imidazol-2-yl, or 1-C₁₋₁₀ alkyl-1H-imidazol-2-yl.

8. A compound in accordance with claim 7 having the formula

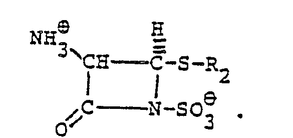

9. A compound in accordance with claim 7 having the formula

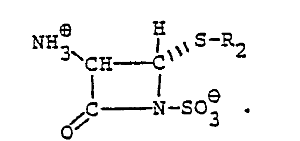

10. Pharmaceutical compositions containing a compound of claim 1 to 6.

## Claims (Claims for the following Contracting State(s) : AT)

1. A process for preparing 4-aryl or heteroarylthio-2-oxo-1-azetidinesulfonic acid salts having the formula I 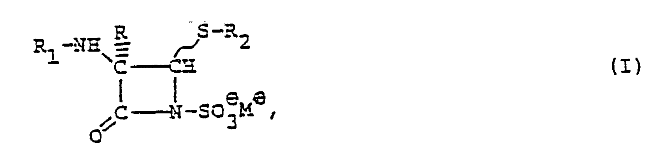 wherein
R is hydrogen or methoxy;
R₁ is an acyl group being known in conventional β-lactam antibiotics;
R₂ is phenyl, optionally substituted with 1, 2 or 3 amino, halogen, hydroxyl, trifluoromethyl, C₁-₄ alkyl or C₁₋₄ alkoxy groups, 1H-tetrazol-5-yl, 1-C₁₋₁₀ alkyl-1H-tetrazol-5-yl, 1,3,4-thiadiazol-2-yl, 5-C₁₋₁₀ alkyl-1,3,4-thiadiazol-2-yl, 1,2,4-thiadiazol-5-yl, 3-C₁₋₁₀ alkyl-1,2,4-thiadiazol-5-yl, 1,3,4-oxadiazol-2-yl, 5-C₁₋₁₀ alkyl-1,3,4-oxadiazol-2-yl, 1,2,4-oxadiazol-5-yl, 3-C₁₋₁₀ alkyl-1,2,4-oxadiazol-5-yl, 2-pyridinyl, 3-pyridinyl, 4-pyridinyl, 2-pyrimidinyl, benzothiazol-2-yl, 4H-1,2,3-triazol-5-yl, 5-C₁₋₁₀ alkyl-4H-1,2,3-triazol-5-yl, 1H-imidazol-2-yl, or 1-C₁₋₁₀ alkyl-1H-imidazol-2-yl; and
M⊕ is hydrogen or a cation, with the proviso that if M⊕ is hydrogen the R₁ group contains a basic function, those compounds being excluded which are described in EP 81 110 154.2 (Publication No. 53 815), namely
sodium (3R,4S)-3-[2-(2-chloroacetamidothiazol-4-yl)-2-methoxyiminoacetamido]-4-phenylthio-2-oxo- azetidine-1-sulfonate,
sodium (3R,4S)-3-[2-(2-aminothiazol-4-yl)-2-methoxyiminoacetamido]-4-phenylthio-2-oxoazetidine-1-sulfonate,
sodium (3R,4S)-3-[2-(2-chloroacetamidothiazol-4-yl)-2-methoxyiminoacetamido]-4-(1-methyl-1H-tetrazol-5-yl)thio-2-oxoazetidine-1-sulfonate,
sodium (3R,4S)-3-[2-(2-aminothiazol-4-yl)-2-methoxyiminoacetamido]-4-(1-methyl-1H-tetrazol-5-yl)thio-2-oxoazetidine-1-sulfonate,
sodium (3R,4R)-3-[2-(2-chloroacetamido-thiazol-4-yl)-2-methoxyiminoacetamido]-4-(1-methyl-1H-tetrazol-5-yl)thio-2-oxoazetidine-1-sulfonate and
sodium (3R,4R)-3-[2-(2-aminothiazol-4-yl)-2-methoxylminoacetamido]-4-(1-methyl-1H-tetrazol-5-yl)thio-2-oxoazetidine-1-sulfonate,
characterized by adding a sulfo group (SO₃⁽⁻⁾) to the 1-position of a compound of the formula 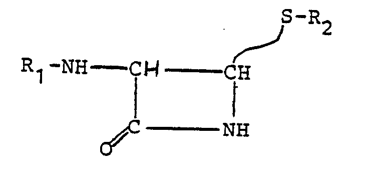 or acylating a zwitterion of the formula VI 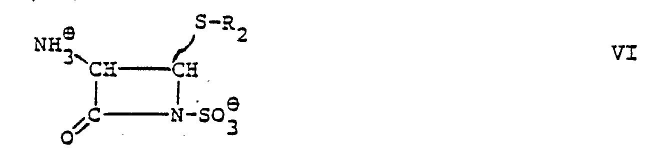 with a carboxylic acid R₁―OH, or with the corresponding acid halide or acid anhydride according to conventional methods, to yield a compound I where R is hydrogen, and, for preparing a compound I where R is methoxy, either
(a) halogenating the amide nitrogen of compound I where R is H to yield in situ an intermediate having the formula VIIa 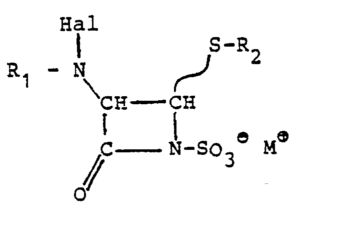 where R₁, R₂ and M have the same meaning as above and Hal represents a halogen atom, and reacting the intermediate Vlla with a methoxylating agent in a manner known per se, or
(b) alkoxylating a compound IVa or IVb 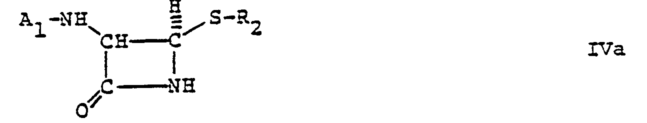 and 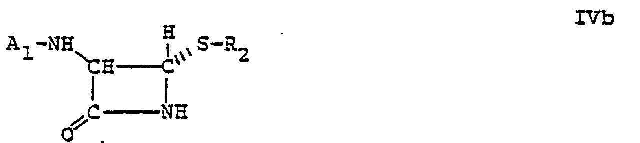 where A₁NH is a usual carbamate substituent (e.g. A, is benzyloxycarbonyl) and R₂ has the same meaning as above, and introducing a sulfo group in the 1-position of the resulting compound, followed by usual deprotection and acylation.

2. A process in accordance with claim 1 for preparing a compound having the formula

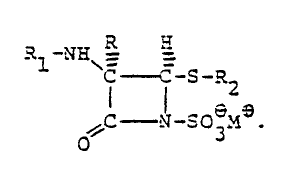

3. A process in accordance with claim 1 for preparing a compound having the formula

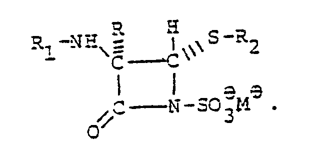

4. A process in accordance with claim 1 for preparing the compound [(3R-trans)(Z)]-[[[(2-amino-4-thiazolyl)methoxyimino]acetyl]amino]-4-[(1-methyl-1H-tetrazol-5-yl)thio]-2-oxo-1-azetidinesulfonic acid, potassium salt.

5. A process in accordance with claim 1 for preparing the compound [(3R-trans)(R*)]-3-[[[[(4-ethyl-2,3-dioxo-1-piperazinyl)carbonyl]amino]phenylacetyl]amino]-2-oxo-4-[(1-methyl-1H-tetrazol-5-yl)thio]-1- azetidinesulfonic acid, potassium salt.

6. A process in accordance with claim 1 for preparing the compound (3R-trans)-4-[(1-methyl-1H-tetrazol-5-yl)thio]-2-oxo-3-[[(phenylmethoxy)carbonyl]amino]-1-azetidinesulfonic acid, potassium salt.

7. A process for preparing a compound having the formula 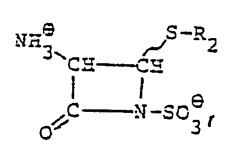 wherein R₂ is phenyl, optionally substituted with 1, 2 or 3 amino, halogen, hydroxyl, difluoromethyl, C₁-₄ alkyl or C₁₋₄ alkoxy groups, 1H-tetrazol-5-yl, 1-C₁₋₁₀ alkyl-1H-tetrazol-5-yl, 1,3,4-thiadiazol-2-yl, 5-C₁₋₁₀ alkyl-1,3,4-thiadiazol-2-yl, 1,2,4-thiadiazol-5-yl, 3-C₁₋₁₀ alkyl-1,2,4-thiadiazol-5-yl, 1,3,4-oxadiazol-2-yl, 5-C₁₋₁₀ alkyl-1,3,4-oxadiazol-2-yl, 1,2,4-oxadiazol-5-yl, 3-C₁₋₁₀ alkyl-1,2,4-oxadiazol-5-yl, 2-pyridinyl, 3-pyridinyl, 4-pyridinyl, 2-pyrimidinyl, benzothiazol-2-yl, 4H-1,2,3-triazol-5-yl, 5-C₁₋₁₀ alkyl-4H-1,2,3-triazol-5-yl, 1H-imidazol-2-yl, or 1-C₁₋₁₀ alkyl-1H-imidazol-2-yl, comprising removing the N-protecting group from a compound of the formula 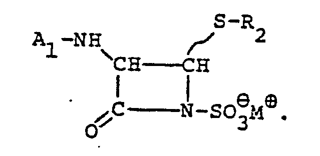 wherein A, is a nitrogen protecting group.

8. A process in accordance with claim 7 for preparing a compound having the formula

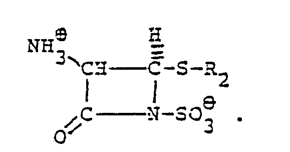

9. A process in accordance with claim 7 for preparing a compound having the formula

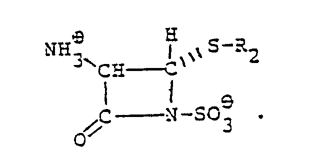

10. A process for preparing a pharmaceutical composition comprising the formulation of a compound produced in accordance with a process of any of claims 1 to 6.

## Claims (Claims for the following Contracting State(s) : GB)

1. 4-Aryl or heteroarylthio-2-oxo-1-azetidinesulfonic acid salts having the formula 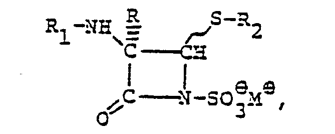 wherein
R is hydrogen or methoxy;
R₁ is an acyl group being known in conventional β-lactam antibiotics;
R₂ is phenyl, optionally substituted with 1, 2 or 3 amino, halogen, hydroxyl, trifluoromethyl, C₁-₄ alkyl or C₁₋₄ alkoxy groups, 1H-tetrazol-5-yl, 1-C₁₋₁₀ alkyl-1H-tetrazol-5-yl, 1,3,4-thiadiazol-2-yl, 5-C₁₋₁₀ alkyl-1,3,4-thiadiazol-2-yl, 1,2,4-thiadiazol-5-yl, 3-C₁₋₁₀ alkyl-1,2,4-thiadiazol-5-yl, 1,3,4-oxadiazol-2-yl, 5-C₁₋₁₀ alkyl-1,3,4-oxadiazol-2-yl, 1,2,4-oxadiazol-5-yl, 3-C₁₋₁₀ alkyl-1,2,4-oxadiazol-5-yl, 2-pyridinyl, 3-pyridinyl, 4-pyridinyl, 2-pyrimidinyl, benzothiazol-2-yl, 4H-1,2,3-triazol-5-yl, 5-C₁₋₁₀ alkyl-4H-1,2,3-triazol-5-yl, 1H-imidazol-2-yl, or 1-C₁₋₁₀ alkyl-1H-imidazol-2-yl; and
M⊕ is hydrogen or a cation, with the proviso that if M⊕ is hydrogen the R₁ group contains a basic function.
2. A compound in accordance with claim 1 having the formula 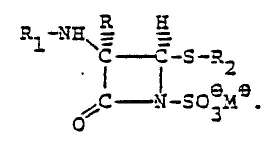
3. A compound in accordance with claim 1 having the formula 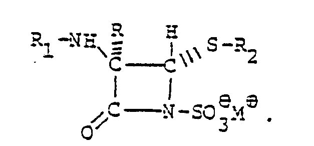
4. The compound in accordance with claim 1 [(3R-trans)(Z)]-[[[(2-amino-4-thiazolyl)methoxy- imino]acetyl]amino]-4-[(1-methyl-1H-tetrazol-5-yl)thio]-2-oxo-1-azetidinesulfonic acid, potassium salt.
5. The compound in accordance with claim 1 [(3R-trans)(R*)]-3-[[[[(4-ethyl-2,3-dioxo-1-piperazinyl)carbonyl]amino]phenylacetyl]amino]-2-oxo-4-[(1-methyl-1H-tetrazol-5-yl)thio]-1-azetidinesulfonic acid, potassium salt.
6. The compound in accordance with claim 1 (3R-trans)-4-[(1-methyl-1H-tetrazol-5-yl)thio]-2-oxo-3-[[(phenylmethoxy)carbonyl]amino]-1-azetidinesulfonic acid, potassium salt.
7. A compound having the formula 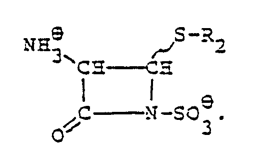 wherein R₂ is phenyl, optionally substituted with 1, 2 or 3 amino, halogen, hydroxyl, difluoromethyl, C₁₋₄ alkyl or C₁₋₄ alkoxy groups, 1H-tetrazol-5-yl, 1-C₁₋₁₀ alkyl-1H-tetrazol-5-yl, 1,3,4-thiadiazol-2-yl, 5-C₁₋₁₀ alkyl-1,3,4-thiadiazol-2-yl, 1,2,4-thiadiazol-5-yl, 3-C₁₋₁₀ alkyl-1,2,4-thiadiazol-5-yl, 1,3,4-oxadiazol-2-yl, 5-C₁₋₁₀ alkyl-1,3,4-oxadiazol-2-yl, 1,2,4-oxadiazol-5-yl, 3-C₁₋₁₀ alkyl-1,2,4-oxadiazol-5-yl, 2-pyridinyl, 3-pyridinyl, 4-pyridinyl, 2-pyrimidinyl, benzothiazol-2-yl, 4H-1,2,3-triazol-5-yl, 5-C₁₋₁₀ alkyl-4H-1,2,3-triazol-5-yl, 1H-imidazol-2-yl, or 1-C₁₋₁₀ alkyl-1H-imidazol-2-yl.
8. A compound in accordance with claim 7 having the formula 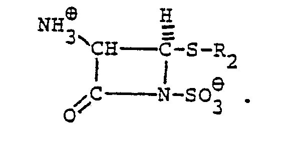
9. A compound in accordance with claim 7 having the formula 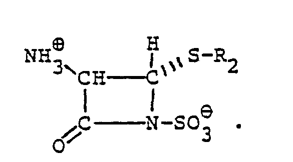
10. Pharmaceutical compositions containing a compound of claim 1 to 6.

## Patentansprüche (Patentansprüche für folgende(n) Vertragsstaat(en) : BE CH DE FR IT LI LU NL SE)

1. 4-Aryl oder -Heteroarylthio-2-oxo-1-azetidin-sulfonsäure-Salze der allgemeinen Formel 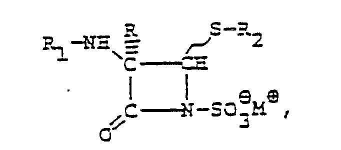 in der
R ein Wasserstoffatom oder eine Methoxygruppe bedeutet;
R₁ einen Acylrest darstellt, der von üblichen β-Lactam-Antibiotika bekannt ist;
R₂ eine Phenylgruppe bedeutet, die gegebenenfalls mit 1, 2 oder 3 Amino-, Halogen-, Hydroxyl-, Trifluormethyl-, C₁₋₄-Alkyl-oder C₁₋₄-Alkoxyresten, 1H-Tetrazol-5-yl-, 1-(C₁₋₁₀-Alkyl)-1H-tetrazol-5-yl-, 1,3,4-Thiadiazol-2-yl-, 5-(C₁-₁₀-Alkyl)-1,3,4-thiadiazol-2-yl-, 1,2,4-Thiadiazol-5-yl-, 3-(C₁₋₁₀-Alkyl)-1,2,4-thiadiazol-5-yl-, 1,3,4-Oxadiazol-2-yl-, 5-(C₁₋₁₀-Alkyl)-1,3,4-oxadiazol-2-yl-, 1,2,4-Oxadiazol-5-yl-, 3-(C₁₋₁₀-Alkyl)-1,2,4-oxadiazol-5-yl-, 2-Pyridinyl-, 3-Pyridinyl-, 4-Pyridinyl-, 2-Pyrimidinyl-, Benzothiazol-2-yl-, 4H-1,2,3-Triazol-5-yl-, 5-(C₁₋₁₀-Alkyl)-4H-1,2,3-triazol-5-yl-, 1H-Imidazol-2-yl- oder 1-(C₁₋₁₀-Alkyl)-1H-imidazol-2-yl-Resten substituiert ist; und
M⊕ ein Wasserstoffatom oder ein Kation bedeutet, mit der Maßgabe, daß, wenn M⊕ ein Wasserstoffatom bedeutet, der Rest R₁ eine basische Funktion enthält, wobei die Verbindungen ausgeschlossen sind, die in der EP 81 11 0154.2 (Veröffentlichungs-Nr. 53 815) beschrieben sind, nämlich
Natrium-(3R,4S)-3-[2-(2-chloracetamidothiazol-4-yl)-2-methoxylminoacetamido]-4-phenylthio-2-oxo- azetidin-1-sulfonat,
Natrium-(3R,4S)-3-[2-(2-aminothiazol-4-yl)-2-methoxylminoacetamido]-4-phenylthio-2-oxoazetidin-1- suifonat,
Natrium-(3R,4S)-3-[2-(2-chloracetamidothiazol-4-yl)-2-methoxylminoacetamido]-4-(1-methyl-1H-tetrazol-5-yl)thio-2-oxoazetidin-1-sulfonat;
Natrium-(3R,4S)-3-[2-(2-aminothiazol-4-yl)-2-methoxylminoacetamido]-4-(1-methyl-1H-tetrazol-5-yl)-thio-2-oxoazetidin-1-sulfonat,
Natrium-(3R,4R)-3-[2-(2-chloracetamido-thiazol-4-yl)-2-methoxylminoacetamido]-4-(1-methyl-1H-tetrazol-5-yl)-thio-2-oxoazetidin-1-sulfonat und
Natrium-(3R,4R)-3-[2-(2-aminothiazol-4-yl)-2-methoxylminoacetamido]-4-(1-methyl-1H-tetrazol-5-yl)-thio-2-oxoazetidin-1-sulfonat.

2. Verbindung nach Anspruch 1 der allgemeinen Formel

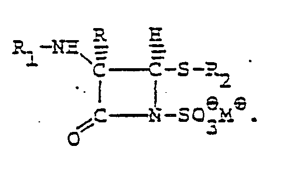

3. Verbindung nach Anspruch 1 der allgemeinen Formel

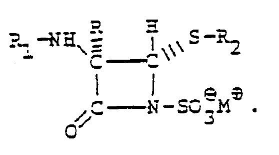

4. Verbindung nach Anspruch 1, nämlich das Kaliumsalz der [(3R-trans)(Z)]-[[[(2-Amino-4-thiazolyl)-methoxyimino]-acetyl]-amino]-4-[(1-methyl-1 H-tetrazol-5-yl)-thio]-2-oxo-1-azetidinsulfonsäure.

5. Verbindung nach Anspruch 1, nämlich das Kaliumsalz der [(3R-trans)(R*)]-3-[[[[(4-äthyl-2,3-dioxo-1-piperazinyl)-carbonyl]-amino]-phenylacetyl]-amino]-2-oxo-4-[(1-methyl-1H-tetrazol-5-yl)-thio]-1-azetidin- sulfonsäure.

6. Verbindung nach Anspruch 1, nämlich das Kaliumsalz der (3R-trans)-4-[(1-Methyl-1 H-tetrazol-5-yl)-thio]-2-oxo-3-[[(phenylmethoxy)-carbonyl]-amino]-1-azetidinsulfonsäure.

7. Verbindung der allgemeinen Formel 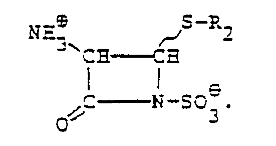 in der R₂ eine Phenylgruppe bedeutet, die gegebenenfalls mit 1, 2 oder 3 Amino-, Halogen-, Hydroxyl-, Trifluormethyl-, C₁₋₄-Alkyl- oder C₁₋₄-Alkoxyresten, 1H-Tetrazol-5-yl-, 1-(C₁₋₁₀-Alkyl)-1H-tetrazol-5-yl-, 1,3,4-Thiadiazol-2-yl-, 5-(C₁₋₁₀-Alkyl)-1,3,4-thiadiazol-2-yl-, 1,2,4-Thiadiazol-5-yl-, 3-(C₁₋₁₀-Alkyl)-1,2,4-thiadiazol-5-yl-, 1,3,4-Oxadiazol-2-yl-, 5-(C₁₋₁₀-Alkyl)-1,3,4-oxadiazol-2-yl-, 1,2,4-Oxadiazol-5-yl-, 3-(C₁₋₁₀-Alkyl)-1,2,4-oxadiazol-5-yl-, 2-Pyridinyl-, 3-Pyridinyl-, 4-Pyridinyl-, 2-Pyrimidinyl-, Benzothiazol-2-yl-, 4H-1,2,3-Triazol-5-yl-, 5-(C₁₋₁₀-Alkyl)-4H-1,2,3-triazol-5-yl-, 1H-Imidazol-2-yl- oder 1-(C₁₋₁₀-Alkyl)-1H-imidazol-2-yl-Resten substituiert ist.

8. Verbindung nach Anspruch 7 der allgemeinen Formel

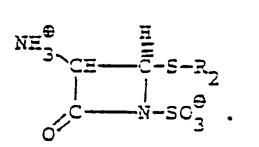

9. Verbindung nach Anspruch 7 der allgemeinen Formel

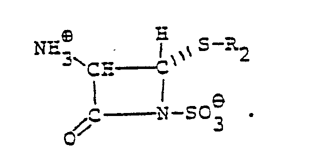

10. Arzneimittel mit einem Gehalt an einer Verbindung der Ansprüche 1 bis 6.

## Patentansprüche (Patentansprüche für folgende(n) Vertragsstaat(en) : AT)

1. Verfahren zur Herstellung von 4-Aryl- oder -Heteroarylthio-2-oxo-1-azetidin-sulfonsäure-Salzen der allgemeinen Formel I 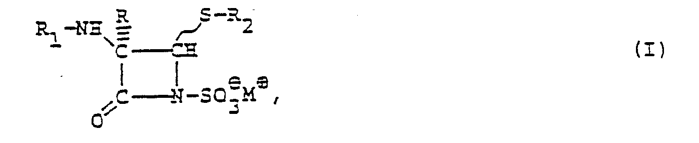 in der
R ein Wasserstoffatom oder eine Methoxygruppe bedeutet;
R₁ einen Acylrest darstellt, der von üblichen β-Lactam-Antibiotika bekannt ist;
R₂ eine Phenylgruppe bedeutet, die gegebenenfalls mit 1, 2 oder 3 Amino-, Halogen-, Hydroxyl-, Trifluormethyl-, C₁₋₄-Alkyl- oder C₁₋₄-Alkoxyresten, 1H-Tetrazol-5-yl-, 1-(C₁₋₁₀-Alkyl)-1H-tetrazol-5-yl-, 1,3,4-Thiadiazol-2-yl-, 5-(C₁-_{1O}-Alkyl)-1,3,4-thiadiazol-2-yl-, 1,2,4-Thiadiazol-5-yl-, 3-(C₁-₁ₒ-Alkyl)-1,2,4-thiadiazol-5-yl-, 1,3,4-Oxadiazol-2-yl-, 5-(C₁₋₁₀-Alkyl)-1,3,4-oxadiazol-2-yl-, 1,2,4-Oxadiazol-5-yl-, 3-(C₁₋₁₀-Alkyl)-1,2,4-Oxadiazol-5-yl-, 2-Pyridinyl-, 3-Pyridinyl-, 4-Pyridinyl-, 2-Pyrimidinyl-, Benzothiazol-2-yl-, 4H-1,2,3-Triazol-5-yl-, 5-(C₁₋₁₀-Alkyl)-4H-1,2,3-triazol-5-yl-, 1H-Imidazol-2-yl- oder 1-(C₁₋₁₀-Alkyl)-1H-imidazol-2-yl-Resten substituiert ist; und
M⊕ ein Wasserstoffatom oder ein Kation bedeutet, mit der Maßgabe, daß, wenn M⊕ ein Wasserstoffatom darstellt, der Rest R₁ eine basische Funktion enthält, wobei die Verbindungen ausgeschlossen sind, die in der EP 81 11 0154.2 (Veröffentlichungs-Nr. 53 815) beschrieben sind, nämlich
Natrium-(3R,4S)-3-[2-(2-chloracetamidothiazol-4-yl)-2-methoxyiminoacetamido]-4-phenylthio-2-oxo- azetidin-1-suifonat,
Natrium-(3R,4S)-3-[2-(2-aminothiazol-4-yl)-2-methoxylminoacetamido]-4-phenylthio-2-oxoazetidin-1-sulfonat,
Natrium-(3R,4S)-3-(2-(2-chloracetamidothiazol-4-yl)-2-methoxylminoacetamido]-4-(1-methyl-1H-tetrazol-5-yl)thio-2-oxoazetidin-1-sulfonat,
Natrium-(3R,4S)-3-[2-(2-aminothiazol-4-yl)-2-methoxylminoacetamido]-4-(1-methyl-1H-tetrazol-5-yl)-thio-2-oxoazetidin-1-sulfonat,
Natrium-(3R,4R)-3-[2-(2-chloracetamido-thiazol-4-yl)-2-methoxylminoacetamido]-4-(1-methyl-1H-tetrazol-5-yl)-thio-2-oxoazetidin-1-sulfonat und
Natrium-(3R,4R)-3-[2-(2-aminothiazol-4-yl)-2-methoxylminoacetamido]-4-(1-methyl-1H-tetrazol-5-yl)-thio-2-oxoazetidin-1-sulfonat.
dadurch gekennzeichnet, daß man eine Sulfogruppe (SO₃⁽⁻⁾) in der 1-Stellung einer Verbindung der allgemeinen Formel 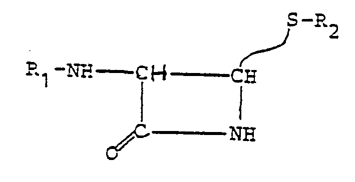 addiert, oder ein Zwitterion der allgemeinen Formel VI 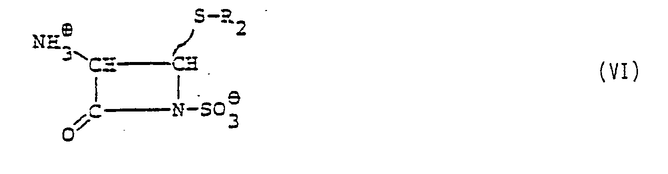 mit einer Carbonsäure R₁-OH oder mit dem entsprechenden Säurehalogenid oder Säureanhydrid gemäß bekannten Verfahren acyliert, wobei man eine Verbindung I erhält, in der R ein Wasserstoffatom darstellt, und zur Herstellung einer Verbindung I, in der R eine Methoxygruppe bedeutet, entweder
(a) den Amidstickstoff der Verbindung I, in der R ein Wasserstoffatom bedeutet, halogeniert, um in situ ein Zwischenprodukt der allgemeinen Formel Vlla 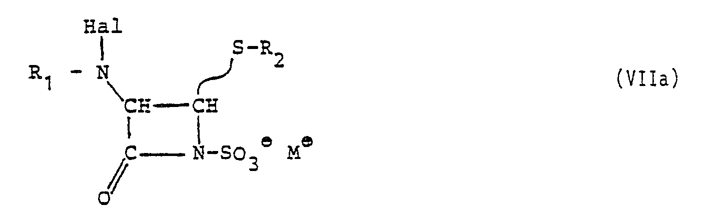 zu erhalten, in der R₁, R₂ und M die vorstehend angegebene Bedeutung haben und Hal ein Halogenatom bedeutet, und das Zwischenprodukt Vlla mit einem Methoxylierungsmittel in an sich bekannter Weise umsetzt, oder
(b) eine Verbindung IVa oder IVb 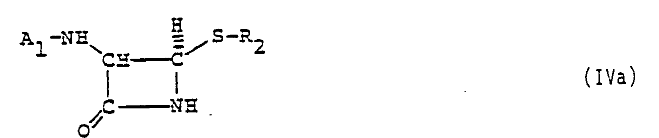 und 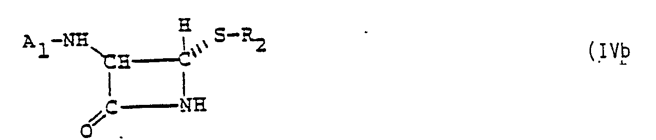 in denen A₁NH einen üblichen Carbamat-Substituenten bedeutet, in dem A₁ beispielsweise Benzyloxycarbonyl darstellt, und R₂ die vorstehend angegebene Bedeutung hat, alkoxyliert und eine Sulfogruppe in der 1-Stellung der erhaltenen Verbindung einführt und anschließend in üblicher Weise die Schutzgruppen entfernt und acyliert.

2. Verfahren nach Anspruch 1 zur Herstellung einer Verbindung der allgemeinen Formel

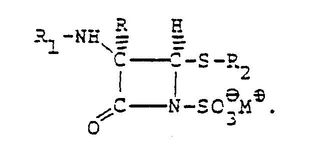

3. Verfahren nach Anspruch 1 zur Herstellung einer Verbindung der allgemeinen Formel

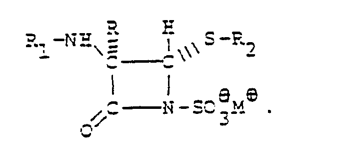

4. Verfahren nach Anspruch 1 zur Herstellung der Verbindung [(3R-trans)(Z)]-[[[(2-Amino-4-thiazolyl)-methoxy-imino]-acetyl]-amino]-4-[(1-methyl-1H-tetrazol-5-yl)-thio]-2-oxo-1-azetidinsulfonsäure-Kaliumsalz.

5. Verfahren nach Anspruch 1 zur Herstellung der Verbindung [(3R-trans)(R*)]-3-[[[[(4-äthyl-2,3-dioxo-1-piperazinyl)-carbonyl]-amino]-phenylacetyl]-amino]-2-oxo-4-[(1-methyl-1H-tetrazol-5-yl)-thio]-1-azetidin- sulfonsäure-Kaliumsalz.

6. Verfahren nach Anspruch 1 zur Herstellung der Verbindung (3R-trans)-4-[(1-Methyl-1H-tetrazol-5-yl)-thio]-2-oxo-3-[[(phenylmethoxy)-carbonyl]-amino]-1-azetidinsulfonsäure-Kaliumsalz.

7. Verfahren zur Herstellung einer Verbindung der allgemeinen Formel 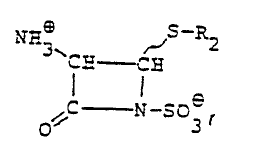 in der R₂ eine Phenylgruppe bedeutet, die gegebenenfalls mit 1, 2 oder 3 Amino-, Halogen-, Hydroxyl-, Trifluormethyl-, C₁₋₄-Alkyl- oder C₁₋₄-Alkoxyresten, 1H-Tetrazol-5-yl-, 1-(C₁₋₁₀-Alkyl)-1H-tetrazol-5-yl-, 1,3,4-Thiadiazol-2-yl-, 5-(C₁₋₁₀-Alkyl)-1,3,4-thiadiazol-2-yl-, 1,2,4-Thiadiazol-5-yl-, 3-(C₁₋₁₀-Alkyl)-1,2,4-thiadiazol-5-yl-, 1,3,4-Oxadiazol-2-yl-, 5-(C₁-_{1O}-Alkyl)-1,3,4-oxadiazol-2-yl-, 1,2,4-Oxadiazol-5-yl-, 3-(C₁-₁₀-Alkyl)-1,2,4-oxadiazol-5-yl-, 2-Pyridinyl-, 3-Pyridinyl-, 4-Pyridinyl-, 2-Pyrimidinyl-, Benzothiazol-2-yl-, 4H-1,2,3-Triazol-5-yl-, 5-(C₁₋₁₀-Alkyl)-4H-1,2,3-triazol-5-yl-, 1H-Imidazol-2-yl- oder 1-(C₁₋₁₀-Alkyl)-1H-imidazol-2-yl-Resten substituiert ist, dadurch gekennzeichnet, daß man die N-Schutzgruppe von einer Verbindung der allgemeinen Formel 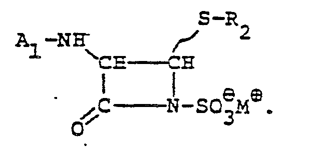 in der A₁ eine Stickstoff-Schutzgruppe bedeutet, entfernt.

8. Verfahren nach Anspruch 7 zur Herstellung einer Verbindung der allgemeinen Formel

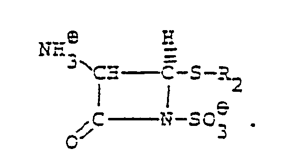

9. Verfahren nach Anspruch 7 zur Herstellung einer Verbindung der allgemeinen Formel

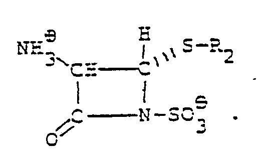

10. Verfahren zur Herstellung eines Arzneimittels, gekennzeichnet durch die Verarbeitung einer Verbindung, die gemäß einem Verfahren nach einem der Ansprüche 1 bis 6 hergestellt wurde.

## Patentansprüche (Patentansprüche für folgende(n) Vertragsstaat(en) : GB)

1. 4-Aryl- oder -Heteroarylthio-2-oxo-1-azetidin-sulfonsäure-Salze der allgemeinen Formel 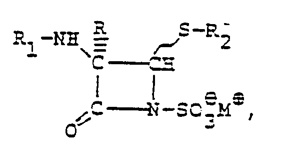 in der
R ein Wasserstoffatom oder eine Methoxygruppe bedeutet;
R₁ einen Acylrest darstellt, der von üblichen β-Lactam-Antibiotika bekannt ist;
R₂ eine Phenylgruppe bedeutet, die gegebenenfalls mit 1, 2 oder 3 Amino-, Halogen-, Hydroxyl-, Trifluormethyl-, C₁₋₄-Alkyl- oder C₁₋₄-Alkoxyresten, 1H-Tetrazol-5-yl-, 1-(C₁₋₁₀-Alkyl)-1H-tetrazol-5-yl-, 1,3,4-Thiadiazol-2-yl-, 5-(C₁₋₁₀-Alkyl)-1,3,4-thiadiazol-2-yl-, 1,2,4-Thiadiazol-5-yl-, 3-(C₁₋₁₀-Alkyl)-1,2,4-thiadiazol-5-yl-, 1,3,4-Oxadiazol-2-yl-, 5-(C₁₋₁₀-Alkyl)-1,3,4-oxadiazol-2-yl-, 1,2,4-Oxadiazol-5-yl-, 3-(C₁₋₁₀-Alkyl)-1,2,4-oxadiazol-5-yl-, 2-Pyridinyl-, 3-Pyridinyl-, 4-Pyridinyl-, 2-Pyrimidinyl-, Benzothiazol-2-yl-, 4H-1,2,3-Triazol-5-yl-, 5-(C₁₋₁₀-Alkyl)-4H-1,2,3-triazol-5-yl-, 1H-Imidazol-2-yl- oder 1-(C₁₋₁₀-Alkyl)-1H-imidazol-2-yl-Resten substituiert ist; und
M⊕ ein Wasserstoffatom oder ein Kation bedeutet, mit der Maßgabe, daß, wenn M⊕ ein Wasserstoffatom darstellt, der Rest R₁ eine basische Funktion enthält.

2. Verbindung nach Anspruch 1 der allgemeinen Formel

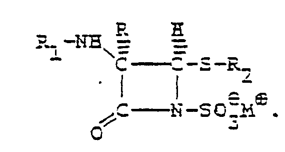

3. Verbindung nach Anspruch 1 der allgemeinen Formel

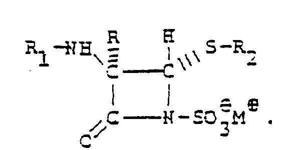

4. Verbindung nach Anspruch 1, nämlich das Kaliumsalz der [(3R-trans)(Z)]-[[[(2-Amino-4-thiazolyl)-methoxylmino]-acetyl]-amino]-4-[(1-methyl-1H-tetrazol-5-yl)-thio]-2-oxo-1-azetidinsulfonsäure.

5. Verbindung nach Anspruch 1, nämlich das Kaliumsalz der [(3R-trans)(R*)]-3-[[[[(4-äthyl-2,3-dioxo-1-piperazinyl)-carbonyl]-amino]-phenylacetyl]-amino]-2-oxo-4-[(1-methyl-1H-tetrazol-5-yl)-thio]-1-azetidin- sulfonsäure.

6. Verbindung nach Anspruch 1, nämlich das Kaliumsalz der (3R-trans)-4-[(1-Methyl-1 H-tetrazol-5-yl)-thio]-2-oxo-3-[[(phenylmethoxy)-carbonyl]-amino]-1-azetidinsulfonsäure.

7. Verbindung der allgemeinen Formel 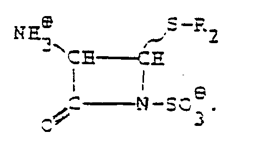 in der R₂ eine Phenylgruppe bedeutet, die gegebenenfalls mit 1, 2 oder 3 Amino-, Halogen-, Hydroxyl-, Trifluormethyl-, C₁₋₄-Alkyl- oder C₁₋₄-Alkoxyresten, 1H-Tetrazol-5-yl-, 1-(C₁₋₁₀-Alkyl)-1H-tetrazol-5-yl-, 1,3,4-Thiadiazol-2-yl-, 5-(C₁₋₁₀-Alkyl)-1,3,4-thiadiazol-2-yl-, 1,2,4-Thiadiazol-5-yl-, 3-(C₁₋₁₀-Alkyl)-1,2,4-thiadiazol-5-yl-, 1,3,4-Oxadiazol-2-yl-, 5-(C₁₋₁₀-Alkyl)-1,3,4-oxadiazol-2-yl-, 1,2,4-Oxadiazol-5-yl-, 3-(C₁₋₁₀-Alkyl)-1,2,4-oxadiazol-5-yl-, 2-Pyridinyl-, 3-Pyridinyl-, 4-Pyridinyl-, 2-Pyrimidinyl-, Benzothiazol-2-yl-, 4H-1,2,3-Triazol-5-yl-, 5-(C₁₋₁₀-Alkyl)-4H-1,2,3-triazol-5-yl-, 1H-Imidazol-2-yl- oder 1-(C₁₋₁₀-Alkyl)-1H-imidazol-2-yl-Resten substituiert ist.

8. Verbindung nach Anspruch 7 der allgemeinen Formel

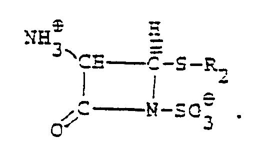

9. Verbindung nach Anspruch 7 der allgemeinen Formel

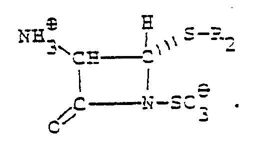

10. Arzneimittel mit einem Gehalt an einer Verbindung der Ansprüche 1 bis 6.

## Revendications (Revendications pour l'(les) Etat(s) contractant(s) suivant(s) : BE CH DE FR IT LI LU NL SE)

1. Sels d'acide 4-aryl ou hétéroarylthio-2-oxo-1-azétidinesulfonique, ayant pour formule 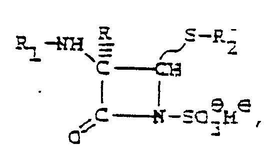 dans laquelle
R est un atome d'hydrogène ou un groupement méthoxy;
R₁ est un radical acyle connu pour les antibiotiques de la série des β-lactames classiques;
R₂ est un radical phényle éventuellement substitué par 1, 2 ou 3 atomes ou groupements amine, halogène, hydroxyle, trifluorométhyle, alkyle en C₁―C₄ ou alcoxy en C₁―C₄, ou bien un radical 1 H-tétrazol-5-yle, 1-alkyl en C₁―C₁₀-1H-tétrazol-5-yle, 1,3,4-thiadiazol-2-yle, 5-alkyl en C₁―C₁₀-1,3,4-thiadiazol-2-yle, 1,2,4-thiadiazol-5-yle, 3-alkyl en C₁―C₁₀-1,2,4-thiadiazol-5-yle, 1,3,4-oxadiazol-2-yle, 5-alkyl en C₁―C₁₀-1,3,4-oxadiazol-2-yle, 1,2,4-oxadiazol-5-yle, 3-alkyl en C₁―C₁₀-1,2,4-oxadiazol-5-yle, 2-pyridinyle, 3-pyridinyle, 4-pyridinyle, 2-pyrimidinyle, benzothiazol-2-yle, 4H-1,2,3-triazol-5-yle, 5-alkyl en C₁―C₁₀-4H-1,2,3-triazol-5-yle, 1H-imidazol-2-yle, ou 1-alkyl en C₁―C₁₀-1H-imidazol-2-yle; et
M⊕ est un atome d'hydrogène ou un cation, à condition que, si M⊕ est un atome d'hydrogène, le groupement R₁ contienne une fonction basique, à l'exclusion des composés qui sont décrits dans EP-81 110 154.2 (publication N° 53.815), à savoir
le (3R,4S)-3-[2-(2-chloracétamidothiazol-4-yl)-2-méthoxylminoacétamido]-4-phénylthio-2-oxo- azétidine-1-sulfonate de sodium,
le (3R,4S)-3-[2-(2-aminothiazol-4-yl)-2-méthoxylminoacétamido]-4-phénylthio-2-oxoazétidine-1-sulfonate de sodium,
le (3R,4S)-3-[2-(2-chloracétamidothiazol-4-yl)-2-méthoxylminoacétamido]-4-(1-méthyl-1H-tétrazol-5-yl)thio-2-oxoazétidine-1-sulfonate de sodium,
le (3R,4S)-3-[2-(2-aminothiazol-4-yl)-2-méthoxylminoacétamido]-4-(1-méthyl-1H-tétrazol-5-yl)thio-2- oxoazétidine-1-suifonate de sodium,
le (3R,4R)-3-[2-(2-chloracétamido-thiazol-4-yl)-2-méthoxyiminoacétamido]-4-(1-méthyl-1H-tétrazol-5-yl)thio-2-oxoazétidine-1-sulfonate de sodium, et
le (3R,4R)-3-[2-(2-aminothiazol-4-yl)-2-méthoxyiminoacétamido]-4-(1-méthyl-1H-tétrazol-5-yl)thio-2- oxoazétidine-1-sulfonate de sodium.

2. Composé selon la revendication 1, ayant pour formule

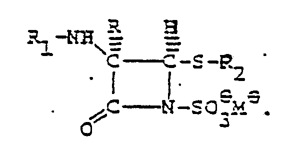

3. Composé selon la revendication 1, ayant pour formule

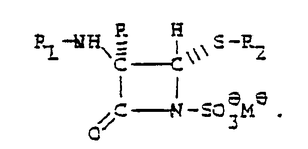

4. Composé selon la revendication 1, qui est le sel de potassium de l'acide [3R-trans)(Z)]-[[[(2-amino-4-thiazolyl)méthoxylmino]acétyl]amino]-4-[(1-méthyl-1H-tétrazol-5-yl)thio]-2-oxo-1-azétidinesulfonique.

5. Composé selon la revendication 1, qui est le sel de potassium de l'acide [(3R-trans)(R^{*})]-3-[[[[(4- éthyl-2,3-dioxo-1-pipérazinyl)carbonyl]amino]phénylacétyl]amino]-2-oxo-4-[(1-méthyl-1H-tétrazol-5-yl)thio]-1-azétidinesulfonique.

6. Composé selon la revendication 1, qui est le sel de potassium de l'acide (3R-trans)-4-[(1-méthyl-1 H-tétrazol-5-yl)thio]-2-oxo-3-[[(phénylméthoxy)carbonyl]amino]-1-azétidinesulfonique.

7. Composé ayant pour formule 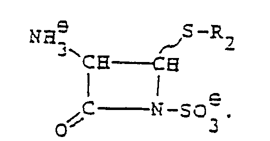 dans laquelle R₂ est un radical phényle éventuellement substitué par 1, 2 ou 3 atomes ou groupements amine, halogène, hydroxyle, trifluorométhyle, alkyle en C₁―C₄ ou alcoxy en C₁―C₄, ou bien un radical 1 H-tétrazol-5-yle, 1-alkyl en C₁―C₁₀-1H-tétrazol-5-yle, 1,3,4-thiadiazol-2-yle, 5-alkyl en C₁―C₁₀-1,3,4-thiadiazol-2-yle, 1,2,4-thiadiazol-5-yle, 3-alkyl en C₁―C₁₀-1,2,4-thiadiazol-5-yle, 1,3,4-oxadiazol-2-yle, 5-alkyl en C₁―C₁₀-1,3,4-oxadiazol-2-yle, 1,2,4-oxadiazol-5-yle, 3-alkyl en C₁―C₁₀-1,2,4-oxadiazol-5-yle, 2-pyridinyle, 3-pyridinyle, 4-pyridinyle, 2-pyrimidinyle, benzothiazol-2-yle, 4H-1,2,3-triazol-5-yle, 5-alkyl en C₁―C₁₀-4H-1,2,3-triazol-5-yle, H-imidazol-2-yle, ou 1-alkyl en C₁―C₁₀-1H-imidazol-2-yle.

8. Composé selon la revendication 7, ayant pour formule

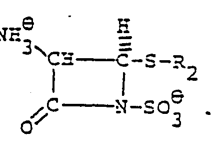

9. Composé selon la revendication 7, ayant pour formule

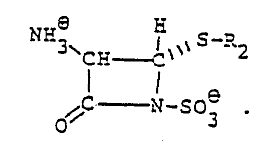

10. Compositions pharmaceutiques contenant un composé selon l'une quelconque des revendications 1 à 6.

## Revendications (Revendications pour l'(les) Etat(s) contractant(s) suivant(s) : AT)

1. Procédé de préparation de sels d'acide 4-aryl ou hétéroarylthio-2-oxo-1-azétidinesulfonique de formule I 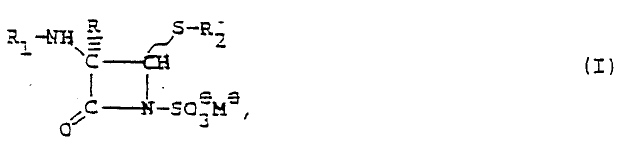 dans laquelle
R est un atome d'hydrogène ou un groupement méthoxy;
R₁ est un radical acyle connu pour les antibiotiques de la série des β-lactames classiques;
R₂ est un radical phényle éventuellement substitué par 1, ou 3 atomes ou groupements amine, halogène, hydroxyle, trifluorométhyle, alkyle en C₁―C₄ ou alcoxy en C₁―C₄, ou bien un radical 1 H-tétrazol-5-yle, 1-alkyl en C₁―C₁₀-1H-tétrazol-5-yle, 1,3,4-thiadiazol-2-yle, 5-alkyl en C₁―C₁₀-1,3,4-thiadiazol-2-yle, 1,2,4-thiadiazol-5-yle, 3-alkyl en C₁―C₁₀-1,2,4-thiadiazol-5-yle, 1,3,4-oxadiazol-2-yle, 5-alkyl en C₁―C₁₀-1,3,4-oxadiazol-2-yle, 1,2,4-oxadiazol-5-yle, 3-alkyl en C₁―C₁₀-1,2,4-oxadiazol-5-yle, 2-pyridinyle, 3-pyridinyle, 4-pyridinyle, 2-pyrimidinyle, benzothiazol-2-yle, 4H-1,2,3-triazol-5-yle, 5-alkyl en C₁―C₁₀-4H-1,2,3-triazol-5-yle, H-imidazol-2-yle, ou 1-alkyl en C₁―C₁₀-1H-imidazol-2-yle; et
M⊕ est un atome d'hydrogène ou un cation, à condition que, si M⊕ est un atome d'hydrogène, le groupement R₁ contienne une fonction basique, à l'exclusion des composés qui sont décrits dans EP-81 110 154.2 (publication N° 53.815), à savoir
le (3R,4S)-3-[2-(2-chloracétamidothiazol-4-yl)-2-méthoxylminoacétamido]-4-phénylthio-2-oxo- azétidine-1-sulfonate de sodium,
le (3R,4S)-3-[2-(2-aminothiazol-4-yl)-2-méthoxylminoacétamido]-4-phénylthio-2-oxoazétidine-1-sulfonate de sodium,
le (3R,4S)-3-[2-(2-chloracétamidothiazol-4-yl)-2-méthoxylminoacétamido]-4-(1-méthyl-1H-tétrazol-5-yl)thio-2-oxoazétidine-1-sulfonate de sodium,
le (3R,4S)-3-[2-(2-aminothiazol-4-yl)-2-méthoxylminoacétamido]-4-(1-méthyl-1H-tétrazol-5-yl)thio-2- oxoazétidine-1-sulfonate de sodium,
le (3R,4R)-3-[2-(2-chloracétamido-thiazol-4-yl)-2-méthoxylminoacétamido]-4-(1-méthyl-1H-tétrazol-5-yl)thio-2-oxoazétidine-1-sulfonate de sodium, et
le (3R,4R)-3-[2-(2-aminothiazol-4-yl)-2-méthoxylminoacétamido]-4-(1-méthyl-1H-tétrazol-5-yl)thio-2- oxoazétidine-1-sulfonate de sodium,
caractérisé en ce qu'on ajoute un groupement sulfo (SO₃⁽⁻⁾) à la position 1 d'un composé de formule 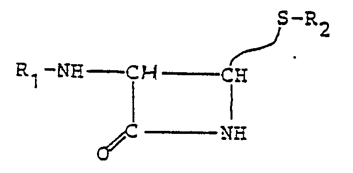 ou bien on acyle un zwitterion de formule VI 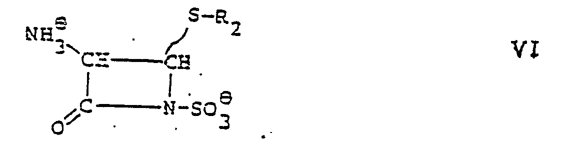 à l'aide d'un acide carboxylique de formule R₁―OH, ou de l'halogénure d'acide ou de l'anhydride d'acide correspondant, selon des procédés classiques, pour obtenir un composé de formule I dans laquelle R est un atome d'hydrogène, et, pour préparer un composé de formule I dans laquelle R est un groupement méthoxy,
(a) ou bien on halogène l'azote amide du composé de formule I dans laquelle R est un atome d'hydrogène pour obtenir, in situ, un intermédiaire de formule Vlla 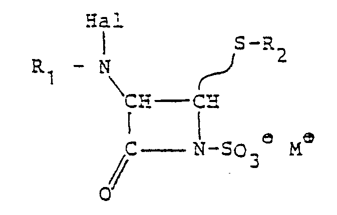 dans laquelle R₁, R₂ et M ont les mêmes significations que ci-dessus et Hal représente un atome d'halogène, et on fait réagir l'intermédiaire de formule Vlla avec un agent méthoxylant, d'une manière connue en soi, ou bien
(b) on alcoxyle un composé de formule IVa ou IVb 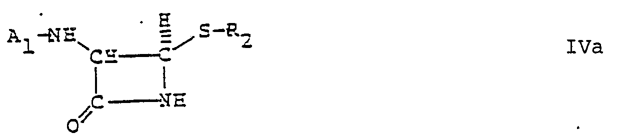 et 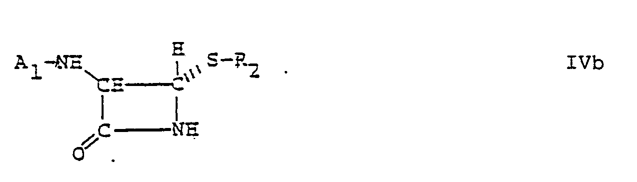 dans lesquelles A₁NH est un substituant carbamate habituel (par exemple A₁ est un radical benzyloxy- carbonyle) et R₂ a la même signification que ci-dessus, et on introduit un groupement sulfo en position 1 du composé résultant, avant de procéder à la suppression de protection et à l'acylation habituelles.

2. Procédé, selon la revendication 1, de préparation d'un composé de formule

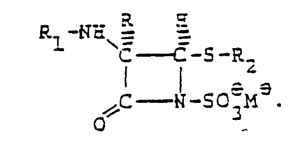

3. Procédé selon la revendication 1, de préparation d'un composé de formule

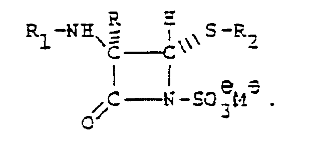

4. Procédé selon la revendication 1, de préparation du sel de potassium de l'acide [(3R-trans)(Z)]-[[[(2-amino-4-thiazolyl)méthoxylmino]acétyl]amino]-4-[(1-méthyl-1H-tétrazol-5-yl)thio]-2-oxo-1-azétidine- suifonique.

5. Procédé, selon la revendication 1, de préparation du sel de potassium de l'acide [(3R-trans)(R^{*})]-3-[[[[(4-éthyl-2,3-dioxo-1-pipérazinyl)carbonyl]amino]phénylacétyl]amino]-2-oxo-4-[(1-méthyl-1H-tétrazol-5-yl)thio]-1-azétidinesulfonique.

6. Procédé, selon la revendication 1, de préparation du sel de potassium de l'acide (3R-trans)-4-[(1-méthyl-1H-tétrazol-5-yl)thio]-2-oxo-3-[[(phénylméthoxy)carbonyl]amino]-1-azétidinesulfonique.

7. Procédé de préparation d'un composé de formule 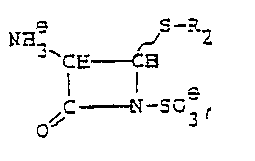 dans laquelle R₂ est un radical phényle éventuellement substitué par 1, 2 ou 3 atomes ou groupements amine, halogène, hydroxyle, trifluorométhyle, alkyle en C₁―C₄ ou alcoxy en C₁―C₄, ou bien un radical 1 H-tétrazol-5-yle, 1-alkyl en C₁―C₁₀-1H-tétrazol-5-yle, 1,3,4-thiadiazol-2-yle, 5-alkyl en C₁―C₁₀-1,3,4-thiadiazol-2-yle, 1,2,4-thiadiazol-5-yle, 3-alkyl en C₁―C₁₀-1,2,4-thiadiazol-5-yle, 1,3,4-oxadiazol-2-yle, 5-alkyl en C₁―C₁₀-1,3,4-oxadiazol-2-yle, 1,2,4-oxadiazol-5-yle, 3-alkyl en C₁―C₁₀-1,2,4-oxadiazol-5-yle, 2-pyridinyle, 3-pyridinyle, 4-pyridinyle, 2-pyrimidinyle, benzothiazol-2-yle, 4H-1,2,3-triazol-5-yle, 5-alkyl en C₁―C₁₀-4H-1,2,3-triazol-5-yle, 1H-imidazol-2-yle, ou 1-alkyl en C₁―C₁₀-1H-imidazol-2-yle, consistant à éliminer le groupement N-protecteur d'un composé de formule 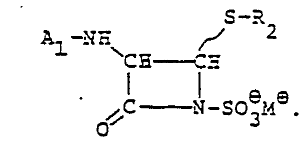 dans laquelle A₁ est un groupement protecteur de l'atome d'azote.

8, Procédé, selon la revendication 7, de préparation d'un composé de formule

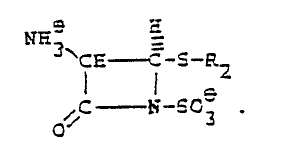

9. Procédé, selon la revendication 7, de préparation d'un composé de formule

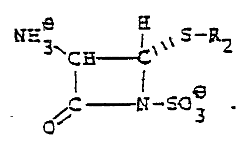

10. Procédé de préparation d'une composition pharmaceutique, consistant à formuler un composé obtenu conformément à un procédé selon l'une quelconque des revendications 1 à 6.

## Revendications (Revendications pour l'(les) Etat(s) contractant(s) suivant(s) : GB)

1. Sels d'acide 4-aryl ou hétéroarylthio-2-oxo-1-azétidinesulfonique, ayant pour formule 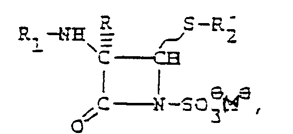 dans laquelle
R est un atome d'hydrogène ou un groupement méthoxy;
R₁ est un radical acyle connu pour les antibiotiques de la série des β-lactames classiques;
R₂ est un radical phényle éventuellement substitué par 1, 2 ou 3 atomes ou groupements amine, halogène, hydroxyle, trifluorométhyle, alkyle en C₁―C₄ ou alcoxy en C₁―C₄, ou bien un radical 1 H-tétrazol-5-yle, 1-alkyl en C₁―C₁₀-1H-tétrazol-5-yle, 1,3,4-thiadiazol-2-yle, 5-alkyl en C₁―C₁₀-1,3,4-thiadiazol-2-yle, 1,2,4-thiadiazol-5-yle, 3-alkyl en C₁―C₁₀-1,2,4-thiadiazol-5-yle, 1,3,4-oxadiazol-2-yle, 5-alkyl en C₁―C₁₀-1,3,4-oxadiazol-2-yle, 1,2,4-oxadiazol-5-yle, 3-alkyl en C₁―C₁₀-1,2,4-oxadiazol-5-yle, 2-pyridinyle, 3-pyridinyle, 4-pyridinyle, 2-pyrimidinyle, benzothiazol-2-yle, 4H-1,2,3-triazol-5-yle, 5-alkyl en C₁―C₁₀-4H-1,2,3-triazol-5-yle, 1H-imidazol-2-yle, ou 1-alkyl en C₁―C₁₀-1H-imidazol-2-yle; et
M⊕ est un atome d'hydrogène ou un cation, à condition que, si M⊕ est un atome d'hydrogène, le groupement R₁ contienne une fonction basique.

2. Composé selon la revendication 1, ayant pour formule

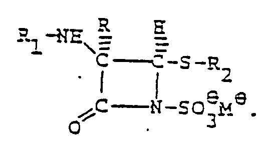

3. Composé selon la revendication 1, ayant pour formule

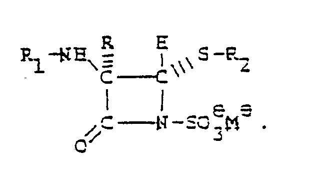

4. Composé selon la revendication 1, qui est le sel de potassium de l'acide [3R-trans)(Z)]-[[[(2-amino-4-thiazolyl)méthoxyimino]acétyl]amino]-4-[(1-méthyl-1H-tétrazol-5-yl)thio]-2-oxo-1-azétidinesulfonique.

5. Composé selon la revendication 1, qui est le sel de potassium de l'acide [(3R-trans)(R*)]-3-[[[[(4- éthyl-2,3-dioxo-1-pipérazinyl)carbonyl]amino]phénylacétyl]amino]-2-oxo-4-[(1-méthyl-1H-tétrazol-5-yl)thio-1-azétidinesulfonique.

6. Composé selon la revendication 1, qui est le sel de potassium de l'acide (3R-trans)-4-[(1-méthyl-1 H-tétrazol-5-yl)thio]-2-oxo-3-[[(phénylméthoxy)carbonyl]amino]-1-azétidinesulfonique.

7. Composé ayant pour formule 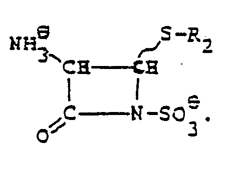 dans laquelle R₂ est un radical phényle éventuellement substitué par 1, 2 ou 3 atomes ou groupements amine, halogène, hydroxyle, trifluorométhyle, alkyle en C,-C₄ ou alcoxy en C₁―C₄, ou bien un radical 1 H-tétrazol-5-yle, 1-alkyl en C₁―C₁₀-1H-tétrazol-5-yle, 1,3,4-thiadiazol-2-yle, 5-alkyl en C₁―C₁₀-1,3,4-thiadiazol-2-yle, 1,2,4-thiadiazol-5-yle, 3-alkyl en C₁―C₁₀-1,2,4-thiadiazol-5-yle, 1,3,4-oxadiazol-2-yle, 5-alkyl en C₁―C₁₀-1,3,4-oxadiazol-2-yle, 1,2,4-oxadiazol-5-yle, 3-alkyl en C₁―C₁₀-1,2,4-oxadiazol-5-yle, 2-pyridinyle, 3-pyridinyle, 4-pyridinyle, 2-pyrimidinyle, benzothiazol-2-yle, 4H-1,2,3-triazol-5-yle, 5-alkyl en C₁―C₁₀-4H-1,2,3-triazol-5-yle, 1H-imidazol-2-yle, ou 1-alkyl en C₁―C₁₀-1H-imidazol-2-yle.

8. Composé selon la revendication 7, ayant pour formule

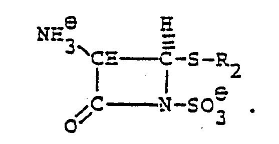

9. Composé selon la revendication 7, ayant pour formule

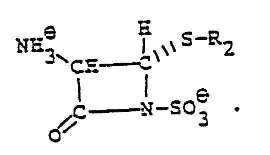

10. Compositions pharmaceutiques contenant un composé selon l'une quelconque des revendications 1 à 6.
